# EUROPEAN PATENT SPECIFICATION

(11) **EP 2 953 556 B1**
(45) Date of publication and mention of the grant of the patent: **06.12.2017**
(21) Application number: 14748471.1
(22) Date of filing: 07.02.2014
(51) Int. Cl.: A61B 17/3215, B65B 1/04, A61B 50/33, A61B 50/20

(54) **SURGICAL INSTRUMENT STRINGER TRAY SYSTEM**
ABLAGESYSTEM FÜR STRINGER FÜR CHIRURGISCHE INSTRUMENTE
SYSTÈME DE PLATEAU À LONGERON POUR INSTRUMENTS CHIRURGICAUX

(30) Priority: 07.02.2013 US 201313761986
(43) Date of publication of application: 16.12.2015
(73) Proprietor: Restore Medical Solutions, Inc., Memphis, Tennessee 38103 (US)
(72) Inventor: RAMKHELAWAN, Ryan, Lawrenceville, Georgia 30044 (US); FLYNN, Shawn, Memphis, Tennessee 38103 (US)
(74) Representative: Emde, Eric
(86) International application number: PCT/US2014/015312
(87) International publication number: WO 2014/124265

(56) References cited:
- US-A- 5 449 069
- US-A1- 2009 093 389
- US-A1- 2009 152 414
- US-A1- 2009 152 414
- US-A1- 2011 114 522
- US-A1- 2011 114 522
- US-A1- 2011 262 250
- US-A1- 2012 234 781

## Description

### TECHNICAL FIELD

The disclosure generally relates to surgical instrument receptacle, organizational system and, more specifically, is related to a stringer and tray system for retaining a collection of surgical instruments for sterilization, transport and storage.

### BACKGROUND

Present day surgical procedures regularly use sets of pre-selected surgical instruments for a specified surgical procedure, such as clamps, hemostat, forceps, scissors, retractors, and the like. These instruments are regularly grouped together to form a set. The set of surgical instruments is stored in a sterilized condition until required for surgery. Prior to sterilization the set of surgical instruments is subject to a time consuming multi-step sorting, identifying, grouping, cleaning and sterilization process. The set of surgical instruments is collected post operation, co-mingled in a wire mesh basket or holed tray for transport to the central sterile processing area. Next, the basket or tray of surgical instruments is placed in a wash sink to brush and manually wash the surgical instruments to remove any foreign debris, such as tissue or dried body fluid. Next, the basket or tray of surgical instruments is transported to and run through an automated washer/decontaminator. Next, the basket or tray of surgical instruments is transported to and emptied out on a sorting table where a technician inspects, counts and sorts each surgical instrument into groupings of instruments for a specified surgical procedure. Next, pivoting or hinged scissor-like surgical instruments are commonly sequentially grouped using a fixed stringer, bar or retaining rod positioned through both of the finger rings or ring handles, and the set is laid or positioned in a basket or tray. Next, the basket or tray of surgical instruments is placed in a sealed container and sealed before entering the sterilization machine. Next, the wrapped or containerized basket or tray of surgical instruments is placed in an industrial sterilization machine/autoclave for sterilization of the surgical instruments. Next, the sealed sterilization container of surgical instruments is stored until transported to an operating area for use as required. When needed the sterilized surgical instruments are transported to the operating room where the surgical instruments are removed from the basket or tray and arranged on a stand or instrument roll in a configuration that enables efficient transfer to a surgeon.

Much time is utilized during the process of cleaning, sorting, counting, and grouping procedure for the assembly and sterilization of surgical instruments. In addition, the onset of infectious diseases has dramatically increased the biohazard risk for medical personnel and central sterile personnel handling post operation cleaning, sorting, counting, and grouping of surgical instruments due to potential contact with sharp surgical instruments, such as needles. Such contact may result in loss of work for recovery, testing for contamination, and/ or a workers compensation claim.

Furthermore, surgical instruments are often damaged when transported, stacked one on the other as well as when the surgical instruments are emptied out on a sorting table for a technician to inspect, count, and sort. Such handling may scratch, bend and may even break the surgical instruments resulting in increased cost to replace such instruments, which are often delicate and expensive. Such damage to the surgical instruments reduces the life expectancy of the surgical instruments resulting in increased medical costs to replace the surgical instruments. Moreover, if such damaged surgical instruments are accidentally returned to the operating room, such surgical procedures may be delayed or cancelled due to non-functioning surgical instruments causing lost revenue for the surgery center and an upset surgical team and patients in queue.

Still further, the environmental impact of the above multi-step process of pre-washing, automated washer/decontaminator, and running the surgical instruments through industrial sterilization machine/autoclave requires large quantities of water, sterilization chemicals and energy.

Yet still further, counting the surgical instruments before and after surgical procedures is particularly important for ensuring that no instruments are left in the patient after the operation procedure has been completed.

Yet still further, one problem with fixed or hinged stringers, bars or retaining rods is that the stringer is not adjustable to string, group or accommodate a variety of surgical instruments nor do such fixed stringers enable adjustment to hold or maintain a variety of surgical instruments in an open position ready for sorting, identifying, grouping, cleaning and sterilization.

Therefore, it is readily apparent that there is a recognizable unmet need for an adjustable surgical instrument stringer with pegs and tray system and method of sterilization that reduces the time spent cleaning, sorting, counting, identifying and grouping surgical instrument, extends the life expectancy of the surgical instruments, provides an adjustable stringer, and decreases the contamination potential of the surgical instruments by maintaining the surgical instruments in a side-by-side open configuration during cleaning and sterilization.

US 2011/114522 A1 discloses a surgical instrument tray system and was used as a basis for the preamble of claim 1.

### SUMMARY

Briefly described, in an example embodiment, the present apparatus overcomes the above-mentioned disadvantages and meets the recognized need for a surgical instrument support tray configured to support a plurality of ring handled surgical instruments. According to the present invention, a surgical instrument support tray is provided as set forth in claim 1. Preferred embodiments of the present invention may be gathered from the dependent claims.

Accordingly, a feature of the surgical instrument support tray is its ability to reduce the time required to identify, clean, sort, count, and group surgical instrument between surgeries.

Another feature of the surgical instrument support tray is its ability to organize, protect and enable thorough cleaning and sterilization of surgical instruments.

Still another feature of the surgical instrument support tray is its ability to accommodate a variety of surgical instruments, such as size, shape, angle of bend, tip type, instruments purpose and the like all in one support tray system.

Yet another feature of the surgical instrument support tray is its ability to rapidly identify the appropriate instrument in the tray during sorting, counting, and grouping of the surgical instrument post-surgery, during pre-wash, pressure washing, sorting, grouping, sterilization or storage for pre-surgical use.

Yet another feature of the surgical instrument support tray is its ability to rapidly identify and select the appropriate surgical instrument during surgery without confusion and lapse of time and to also identify all surgical instruments post-surgery.

Yet another feature of the surgical instrument support tray is its ability to reduce inefficiency and waste in turning around sterile surgical instruments.

Yet another feature of the surgical instrument support tray is its ability to decrease the loss of surgical instruments, decrease the time to count the surgical instruments before and after surgical procedures, and decrease assembly time of surgical instrument sets.

Yet another feature of the surgical instrument support tray is its ability to reduce surgical instrument inventory or reduce the need for additional capital expenditures due to increased surgical volume as a result of more efficient processing time.

Yet another feature of the surgical instrument support tray is its ability to enable a uniform cleaning process for reducing the potential for surgical site infection.

Yet another feature of the surgical instrument support tray is its ability to reduce the occurrences of malfunctions, delays or cancellations during the surgical procedure due to improperly inspected or cleaned surgical instruments.

Yet another feature of the surgical instrument support tray is its ability to reduce the capital budget for repairs associated with care and handling of surgical instruments.

Yet another feature of the surgical instrument support tray is its ability to integrate the surgical instrument support tray as the tray top of box like container.

Yet another feature of the surgical instrument support tray is its ability to reduce the number of sharps being sent to central sterile and the reduction of accidents associated with needle/sharps injuries.

Yet another feature of the surgical instrument support tray is its ability to inventory surgical blades and needles after being disengaged therefrom.

Yet another feature of the surgical instrument support tray is its ability to color coordinate a group of trays within a surgical service for easy identification for specific surgical specialties.

Yet another feature of the surgical instrument support tray is its ability to reduce surgical instrument damage when transporting, stacking, sorting, inspecting, counting, and/or when empting out on a sorting table for a technician to inspect, count, and sort. Such handling may scratch, bend and may even break the surgical instruments resulting in increased cost to replace such instruments, which are often delicate and expensive. Such damage to the surgical instruments reduces the life expectancy of the surgical instruments resulting in increased medical costs to replace the surgical instruments.

Yet another feature of the surgical instrument support tray is its ability to sort, identify, group, clean, and sterilize and to further prevent damaged or improperly cleaned surgical instruments from accidentally being returned to the operating room, where such surgical procedures may be delayed or cancelled due to non-functioning surgical instruments and further causing lost revenue for the surgery center.

Yet another feature of the surgical instrument support tray is its ability to reduce the environmental impact of the multi-step process of pre-washing, automated washer/decontaminator, and running the surgical instruments through industrial sterilization machine/autoclave to reduce the requirements for large quantities of water, sterilization chemicals and energy.

Yet another feature of the surgical instrument support tray is its ability to provide an adjustable stringer to group or accommodate a variety of surgical instruments and provide adjustment to hold or maintain a variety of surgical instruments in an open position ready for sorting, identifying, grouping, cleaning and sterilization.

Yet another feature of the surgical instrument support tray is its ability to provide one or more pegs to be utilized to affix stringer and/or a group of surgical instrument bound together by stringer to the tray or tray top.

Yet another feature of the surgical instrument support tray is its ability to provide a linear adjustment or latch and release connector between sections.

Yet another feature of the surgical instrument support tray is its ability to provide a stringer that may be positioned in or across valleys on tray top by adjusting the stringer wherein the surgical instruments may be retained in parallel or nonparallel to the valleys, evenly spaced and an open jaw position by the surgical instrument support tray.

Yet another feature of the surgical instrument support tray is its ability to provide a stringer that accommodates shorter shanked surgical instrument sets and longer shanked surgical instrument sets, on one stringer in a wide open position for better cleaning and sterilization purposes and to enable easy identification, pressure washing, sorting, counting, and grouping of surgical instruments.

These and other features of the surgical instrument support tray will become more apparent to one skilled in the art from the following Brief Description of the Drawings, Detailed Drawings, Detailed Description and Claims when read in light of the accompanying drawings.

### BRIEF DESCRIPTION OF THE DRAWINGS

The present surgical instrument support tray will be better understood by reading the Detailed Description of the embodiments with reference to the accompanying drawings, in which like reference to numerals denote similar structures and refer to like elements throughout, and in which:
**FIG. 1** is a perspective view of an example stringer;
**FIG. 2** is a top view, side view and bottom view of the stringer of **FIG. 1****;**
**FIG. 3** is an exploded perspective view of the stringer of **FIG. 1****;**
**FIG. 4****.****1** is top view of an example surgical instrument for storage in the surgical instrument support tray system shown herein;

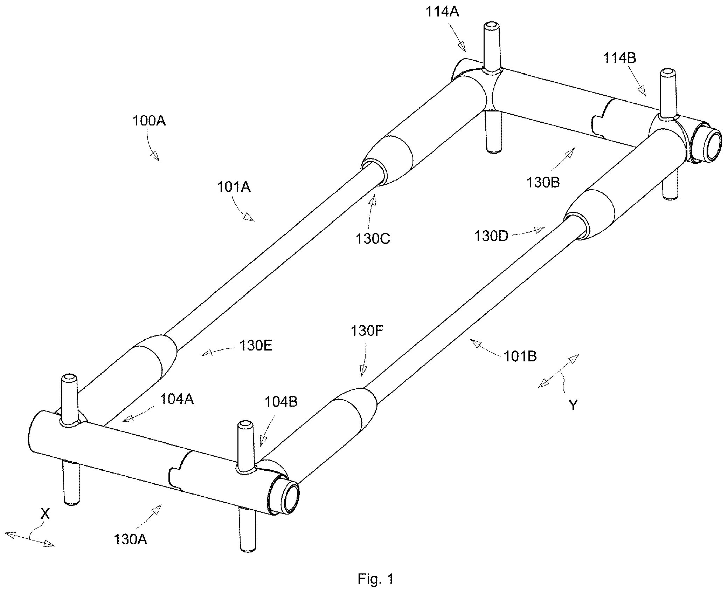

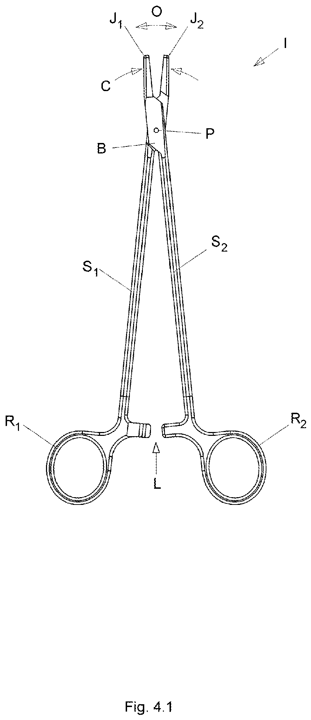

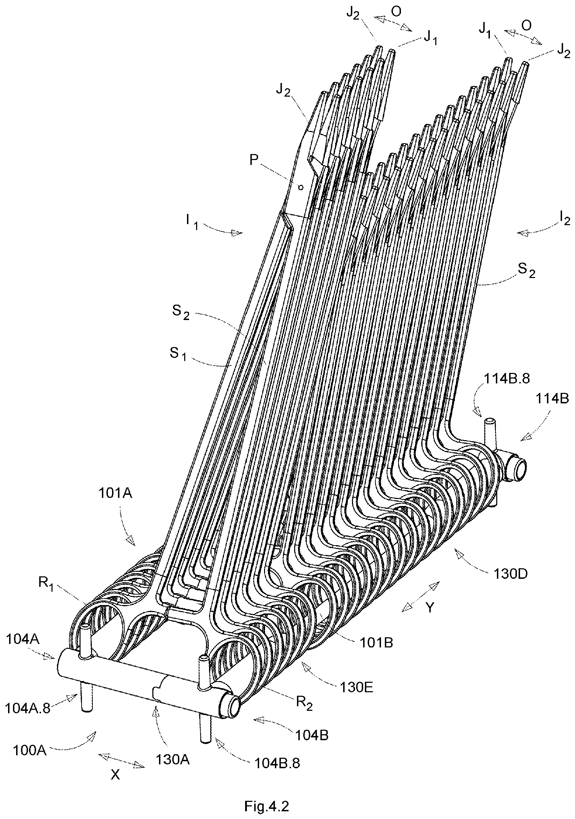

**FIG. 4****.****2** is perspective view of a plurality of surgical instruments of **FIG. 4****.****1** shown strung together with stringer of **FIG. 1****;**

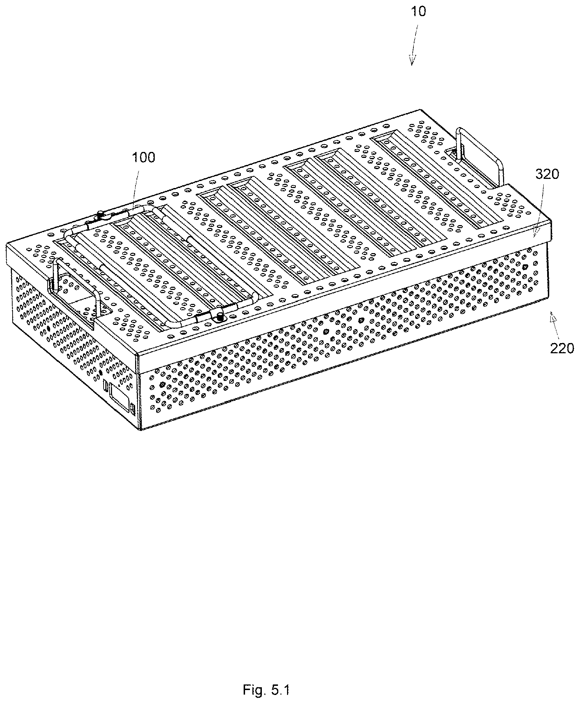

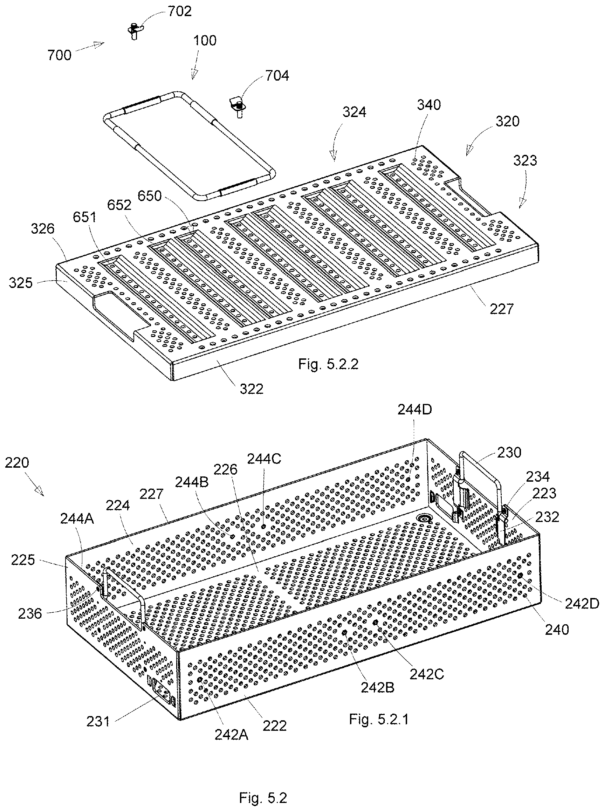

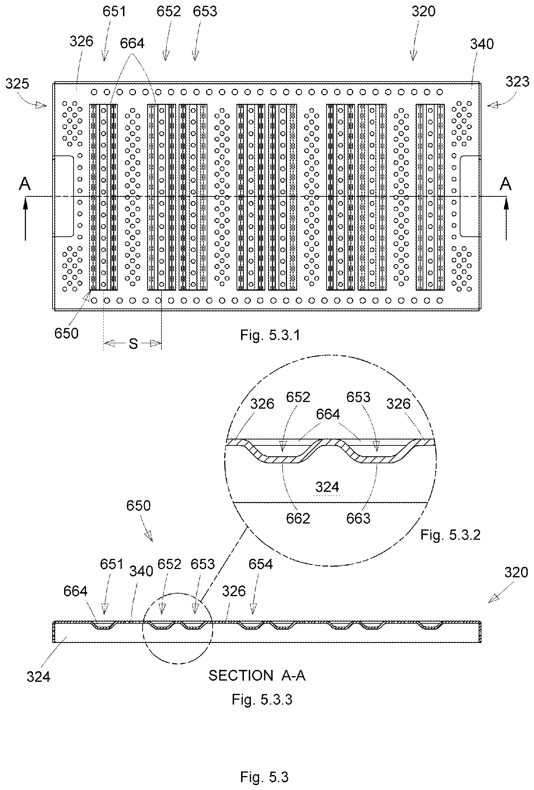

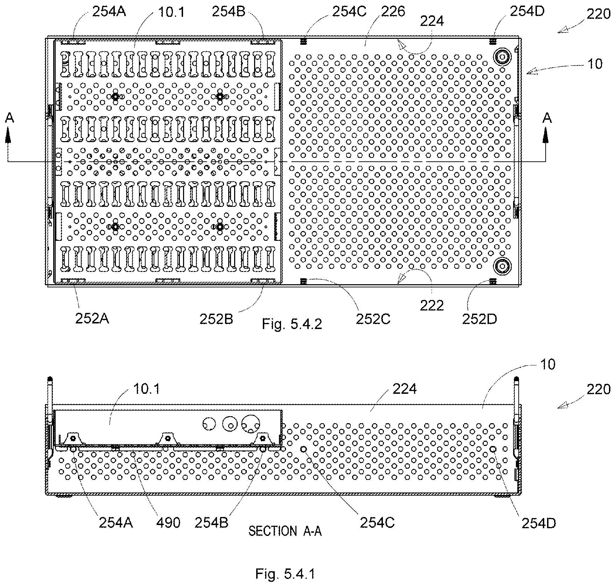

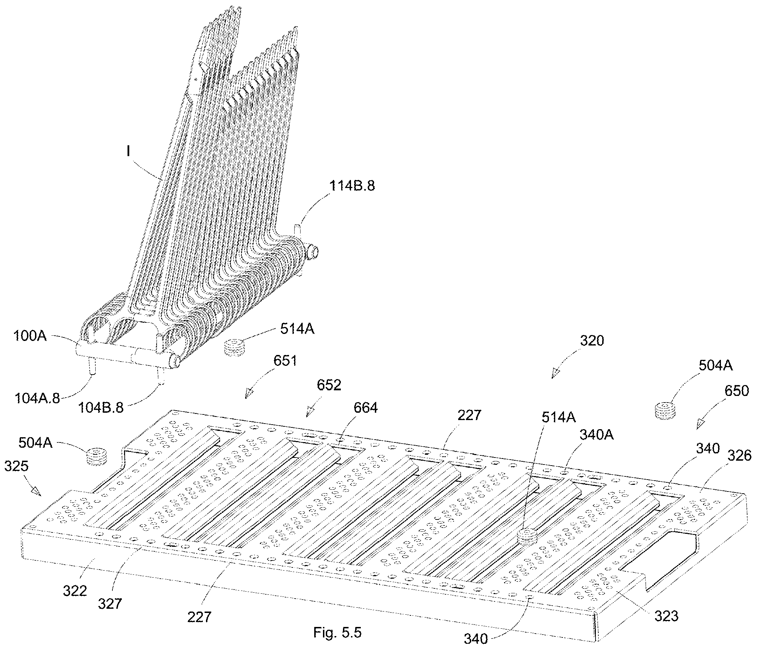

**FIG. 5****.****1** is perspective view of an example embodiment box like tray bottom and lid surgical instrument support tray system with exemplary adjustable stringer position thereon;
**FIG. 5****.****2** is an exploded perspective view of an example embodiment box like tray bottom and lid surgical instrument support tray system with exemplary adjustable stringer;
**FIG. 5****.****3** is a top and cross sectional view of an example embodiment box lid and surgical instrument support system;
**FIG. 5****.****4** is a top and cross sectional view of an example embodiment box like tray bottom and internal small box support system;
**FIG. 5.5** is perspective view of an example embodiment box like lid or top for a surgical instrument support tray system with exemplary stringer of **FIG. 1** positioned thereabove;

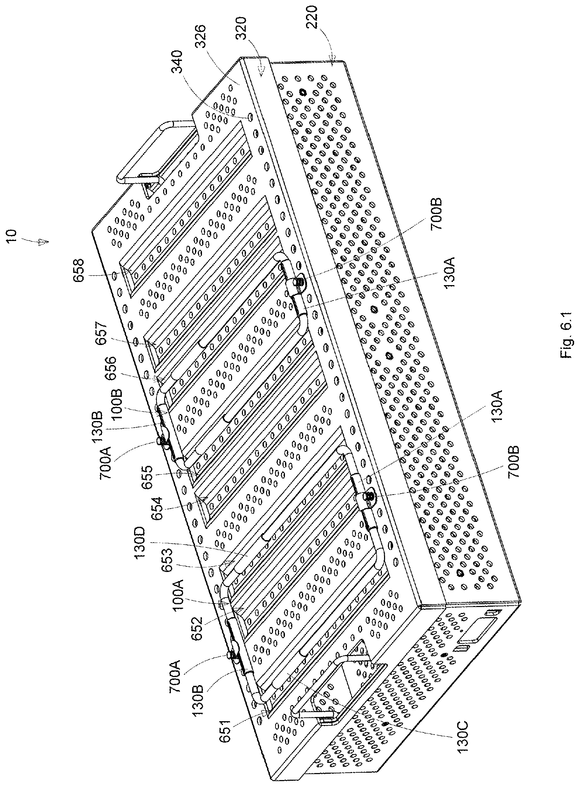

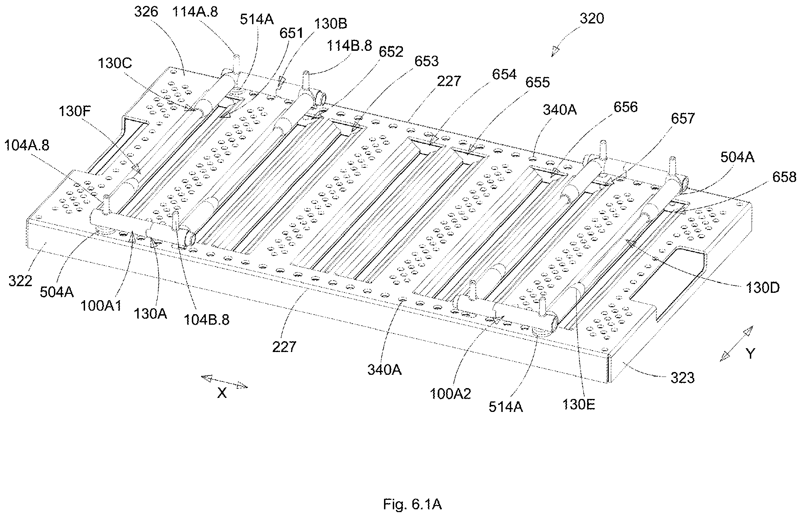

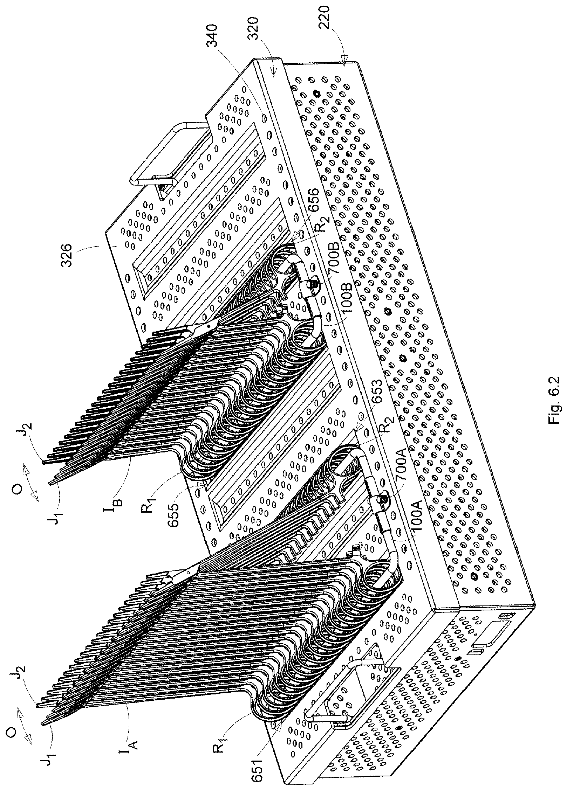

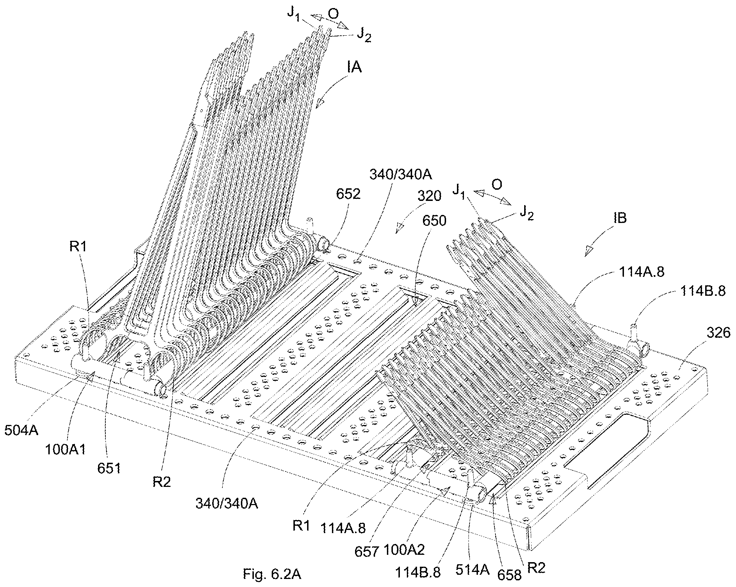

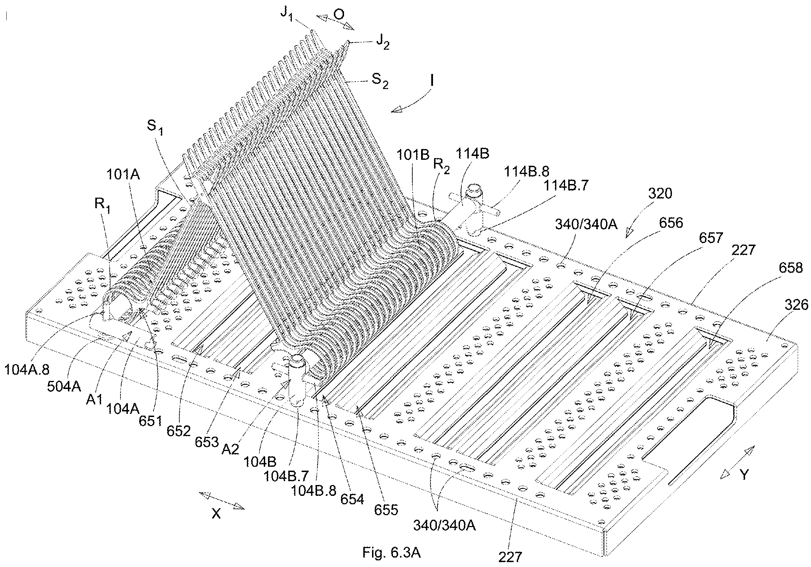

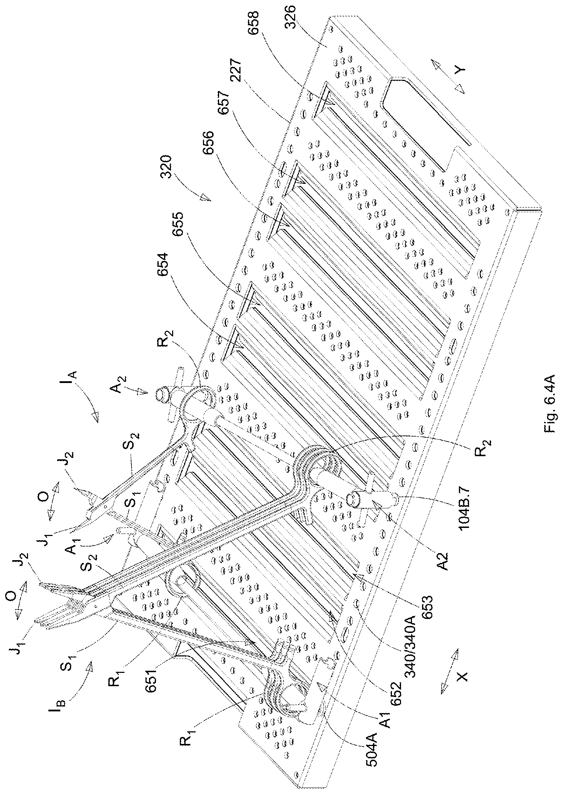

**FIG. 6****.****1** is a perspective view of an example embodiment of a surgical instrument support tray system with exemplary adjustable stringers shown in two positions affixed to the top or lid of the surgical instrument support tray system;
**FIG. 6.1A** **is** a perspective view of an example embodiment of a box like lid or top of a surgical instrument support tray system with exemplary stringers of **FIG. 1** shown in two positions releasably affixed to the top or lid of the surgical instrument support tray system;
**FIG. 6****.****2** is a perspective view of an example embodiment of a surgical instrument support tray system with a plurality of surgical instrument sets held by exemplary adjustable stringers to the top of the surgical instrument support tray system;
**FIG. 6.2A** is a perspective view of an example embodiment of a lid or top for a surgical instrument support tray system with a plurality of surgical instrument sets held by exemplary stringers of **FIG. 1** to the top of the surgical instrument support tray system;
**FIG. 6.3A** is a perspective view of an example embodiment of a lid or top for a surgical instrument support tray system with a plurality of surgical instrument sets held by exemplary unlatched stringer of **FIG. 1** to the top of the surgical instrument support tray system with ring handles positioned parallel to valleys formed in the lid or top;
**FIG. 6.4A** is a perspective view of an example embodiment of a lid or top for a surgical instrument support tray system with a plurality of surgical instrument sets held by exemplary unlatched stringer of **FIG. 1** to the top of the surgical instrument support tray system with ring handles positioned nonparallel to valleys formed in the lid or top;

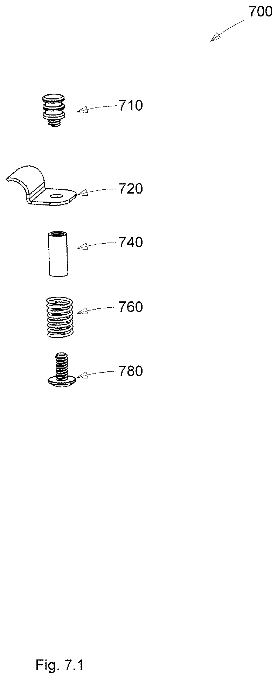

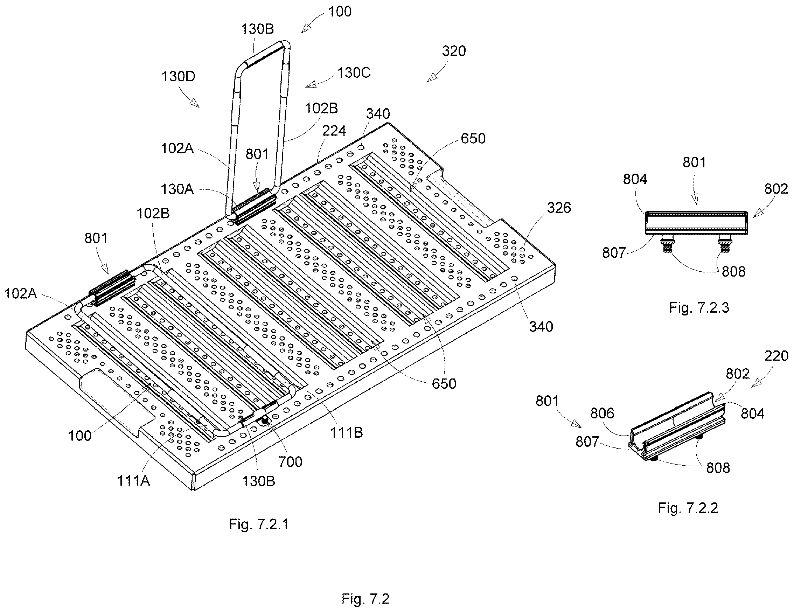

**FIG. 7****.****1** is an exploded perspective view of the attachment device of **FIG. 5****.****2.2****;**
**FIG. 7****.****2** is a perspective view of an alternate exemplary attachment device of **FIG. 7****.****1****;** and

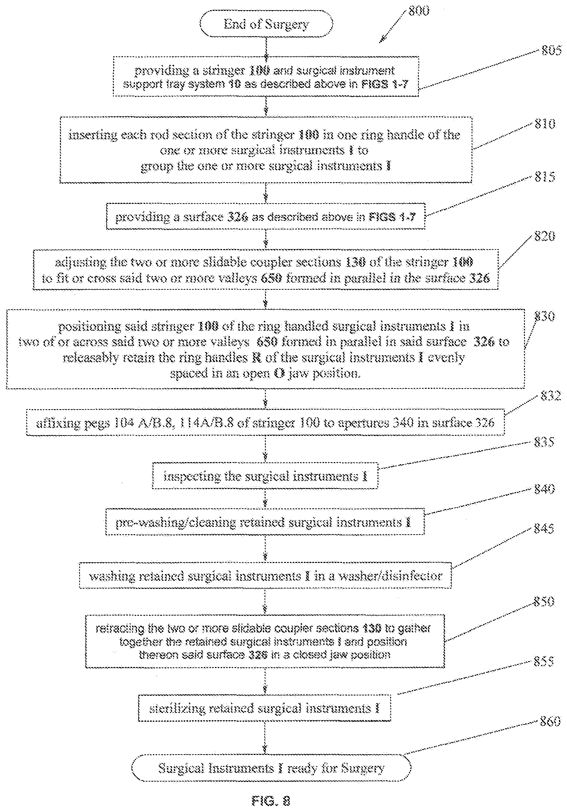

**FIG. 8** is a flow diagram of a method of sorting, identifying, grouping, counting, cleaning, pressure washing, sterilizing, and storing surgical instruments prior to surgical use.

It is to be noted that the drawings presented are intended solely for the purpose of illustration and that they are, therefore, neither desired nor intended to limit the disclosure to any or all of the exact details of construction shown, except insofar as they may be deemed essential to the claimed invention.

### DETAILED DESCRIPTION

In describing the exemplary embodiments of the present disclosure as illustrated in **FIGS. 1-8** specific terminology is employed for the sake of clarity. The present disclosure, however, is not intended to be limited to the specific terminology so selected, and it is to be understood that each specific element includes all technical equivalents that operate in a similar manner to accomplish similar functions. Embodiments of the claims may, however, be embodied in many different forms and should not be construed to be limited to the embodiments set forth herein. The examples set forth herein are non-limiting examples, and are merely examples among other possible examples.

Referring now to **FIG. 1** there is illustrated a perspective view of an exemplary adjustable or fixed bar or retaining rod, such as stringer **100A.** Preferably, stringer **100A** includes one or more bars, tubes, conduits or the like, such as rod **101A/B,** one or more bends, corners, angles, right angle, acute angle, curves, or turns, such as angle sections **104A/B** or **114A/B,** and one or more expand and contract sections, slidable sections, latchable sections, telescope, or releasable sections, such as connector **130A/B/C/D/E/F.** It is contemplated herein that other configurations of connector **130A/B/C/D/E/F** known to one of ordinary skill in the art, such as other connector configurations, multi segment connector, telescopic slidable connector sections or the like to accommodate larger surgical instruments **I** are included herein. It is further contemplated herein that rod **101A/B** and angle sections **104A/B** or **114A/B** may be formed as a plurality of pieces or parts for assembly as a whole stringer **100A** or as one or more single elements for assembly as a whole stringer **100A.** Stringer **100A** is preferably configured as an adjustable rectangular configuration with adjustments in one or more axis, such as x axis **X** and/or y axis **Y** via connector **130A/B/C/D/E/F;** however, other configurations, such as square, trapezoid, trapezium, or the like and other adjustments or adjustment mechanisms in and between axis are contemplated herein.

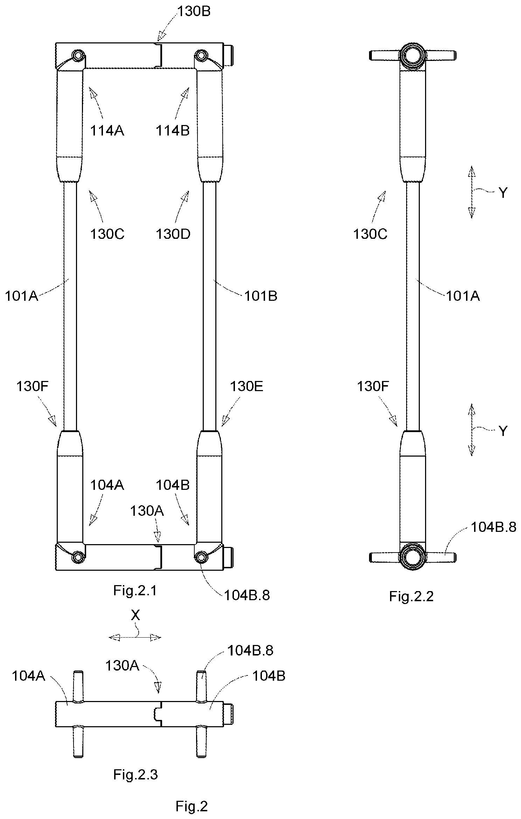

Referring now to **FIG. 2** there is illustrated a top view, side view and bottom view of stringer **100A.** **FIG. 2****.****1** further illustrates the rectangular configuration of rods **101A/B** and **111A/B,** angle sections **104A/B** and **114A/B,** and connector **130A/B/C/D/E/F.** **FIG. 2.2** further illustrates the linear adjustment capability in the y axis **Y** between rod **101A** and angle sections **104A** and/or **114A** via connector section **130C/F** configured to enable releasable extension and retraction of rod **101A** in and out therein of connector **130C/F.** It is contemplated herein that rod **101B** may be configured to enable releasable extension and retraction of rod **101B** in and out therein of connector section **130D/E.** **FIG. 2****.****3** further illustrates the linear adjustment or latch and release capability in the x axis **X** between angle sections **104A** and **104B** via connector **130A.** It is contemplated herein that connector **130B** likewise may be configured to enable the linear adjustment or latch and release capability in the x axis **X** between angle sections **114A** and **114B** via connector **130B.**

Stringer **100A** is preferably formed of a suitable surgical material, such as stainless steel, aluminum, metal, metal alloys, shape memory alloys, carbon fibers, nylon, ceramic or the like, capable of providing structure whether as a solid or hollow stringer **100A.** Preferably, the material includes other suitable characteristics, such as durability, rigidity, stain-resistance, bacteria-resistant, light weight, chemical inertness, oxidation resistance, ease of workability, or other beneficial characteristic understood by one skilled in the art. Moreover, stringer **100A** is preferably configured having a cross-sectional circular diameter; however, other configurations, such as square or the like, are contemplated herein. Stringer **100A** is preferably solid for the purpose of preventing any interior surface capable of colonizing bacteria, viruses or other infectious diseases and difficult for sterilization chemicals to reach; however, a hollow interior is contemplated herein if such interior is sealed or alternatively if a plurality of holes or apertures are present to enable sterilization of the interior surface of stringer **100A.**

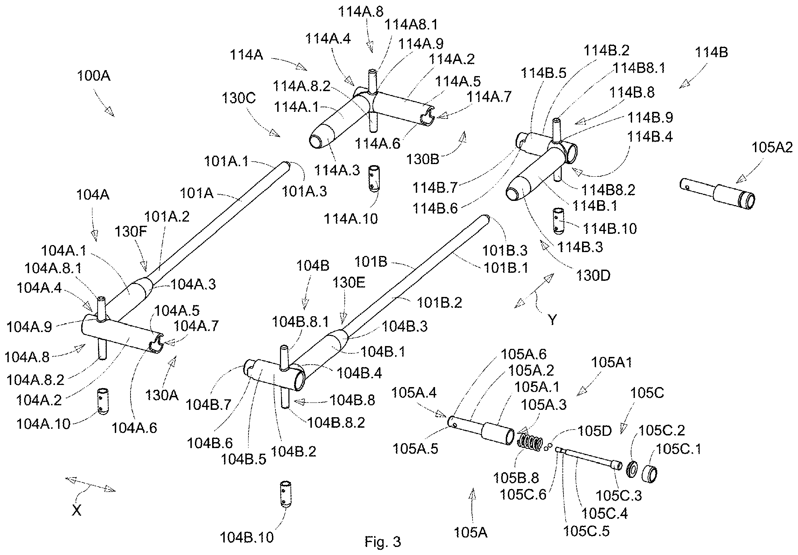

Referring now to **FIG. 3** there is illustrated an exploded view of rod **101A,** rod **101B,** angle sections **104A/B** or **114A/B,** and connectors **130A/B/C/D/E/F** of stringer **100A.** Preferably rod **101A** includes first rod end **101A.1** and second rod end **101A.2.** First rod end **101A.1** preferably includes a solid end, rounded end, pointed end, or cover, such as rod end cap **101A.3.** Second rod end **101A.2** preferably transitions to or is releasably connected to angle section **104A.** Preferably rod **101B** includes first rod end **101B.1** and second rod end **101B.2.** First rod end **101B.1** preferably includes a solid end, rounded end, pointed end, or cover, such as rod end cap **101B.3.** Second rod end **101B.2** preferably transitions to or is releasably connected to angle section **104B.**

Preferably angle section **104A** includes first connector section **104A.1** configured approximately perpendicular, curved, or right angled to second connector section **104A.2** and both are preferably adjoined to one another at a common intersection **104A.4.** First connector section **104A.1** preferably includes a transition end or tapered end, such as first connector end **104A.3** configured to releasably connect second rod end **101A.2** of rod **101A** thereto first connector section **104A.1** of angle section **104A.** It is recognized herein that connector **130F** may be an adhesive, compression fitting, mechanical fitting, weld, single molded component of rod **101A** and angle section **104A** or the like. It is alternatively contemplated herein that connector **130F** may preferably comprise a fixed connection therebetween first connector end **104A.3** of first connector section **104A.1** and second rod end **101A.2** of rod **101A.** Moreover, second connector section **104A.2** of angle section **104A** preferably includes a transition end or fitted end, such as second connector end **104A.5** configured to releasably connect second connector section **104A.2** of angle section **104A** thereto angle section **104B.** Preferably, second connector end **104A.5** includes receptacle **104A.7** and further includes first edge **104A.6** configured preferably to match, fit, (notched-slotted) and/or receive angle section **104B** to aid in the proper alignment of angle sections **104A/B** and to maintain rods **101A/B** in alignment or parallel with each other. It is contemplated herein that receptacle **104A.7** and/or first edge **104A.6** may be alternatively configured to accommodate and/or releasably connect second connector section **104A.2** of angle section **104A** thereto angle section **104B.**

Preferably, second connector section **104A.2** of angle section **104A** includes a pin, leg, support, or hanger, such as first peg **104A.8** configured approximately vertical, perpendicular or right angled to second connector section **104A.2** and extends above (first peg **104A.8.1**) and/or below (first peg **104A.8.2**) second connector section **104A.2.** First peg **104A.8** is preferably inserted through conduit **104A.9** configured through second connector section **104A.2** or may have been formed as a part of second connector section **104A.2** or otherwise therein angle section **104A.** It is contemplated herein that first peg **104A.8** may alternatively have likewise been positioned or formed as a part of first connector section **104A.1.** Furthermore, first peg **104A.8** may be configured with grommet or cover **104A.10** to releasably affix or hold first peg **104A.8** thereto apertures **340** formed tray top **320.**

It is contemplated herein that angle section **104A** may be formed or molded as one piece including first connector section **104A.1,** second connector section **104A.2,** and first peg **104A.8.**

Preferably angle section **104B** includes first connector section **104B.1** configured approximately perpendicular, curved, or right angled to second connector section **104B.2** and both are preferably adjoined to one another at a common intersection **104B.4.** First connector section **104B.1** preferably includes a transition end or tapered end, such as first connector end **104B.3** configured to releasably connect second rod end **101B.2** of rod **101B** thereto first connector section **104B.1** of angle section **104B.** It is recognized herein that connector **130E** may be an adhesive, compression fitting, weld, mechanical connector, single molded component of rod **101B** and angle section **104B** or the like. It is alternatively contemplated herein that connector **130E** may preferably comprise a fixed connection therebetween such as first connector end **104B.3** of first connector section **104B.1** and second rod end **101B.2** of rod **101B.** Moreover, second connector section **104B.2** of angle section **104B** preferably includes a transition end or fitted end, such as second connector end **104B.5** configured to releasably connect second connector section **104B.2** of angle section **104B** thereto angle section **104A.** Preferably, second connector end **104B.5** includes protrusion **104B.7** and further includes first edge **104B.6** configured preferably to match, fit, (notched-slotted) first edge **104A.6** and/or insert therein receptacle **104A.7** of angle section **104A** to aid in the proper alignment of angle sections **104A/B** and to maintain rods **101A/B** in alignment or parallel with each other. It is contemplated herein that protrusion **104B.7** and/or first edge **104B.6** may be alternatively configured to accommodate and/or releasably connect second connector section **104B.2** of angle section **104B** thereto angle section **104A.**

Preferably, second connector section **104B.2** of angle section **104B** includes a pin, leg, support, or hanger, such as second peg **104B.8** configured approximately vertical, perpendicular or right angled to second connector section **104B.2** and extends above (second peg **104B.8.1**) and/or below (second peg **104B.8.2)** second connector section **104B.2.** Second peg **104B.8** is preferably inserted through conduit **104B.9** configured through second connector section **104B.2** or may have been formed as a part of second connector section **1048.2** or angle section **104B.** It is contemplated herein that second peg **104B.8** may alternatively have been positioned or formed as a part of first connector section **104B.1.** Furthermore, second peg **104B.8** may be configured with grommet or cover **104B.10** to releasably affix or hold second peg **104B.8** thereto apertures **340** formed tray top **320.**

It is contemplated herein that angle section **104B** may be formed or molded as one piece including first connector section **104B.1,** second connector section **104B.2,** and second peg **104B.8.**

Preferably angle section **114A** includes first connector section **114A.1** configured approximately perpendicular, curved, or right angled to second connector section **114A.2** and both are preferably adjoined to one another at a common intersection **114A.4.** First connector section **114A.1** preferably includes a transition end or tapered end, such as first connector end **114A.3** configured to releasably and slidably connect first rod end **101A.1** of rod **101A** thereto first connector section **114A.1** of angle section **114A.** It is recognized herein that connector **130C** may be an adhesive, compression fitting, weld, mechanical connector, single molded component of rod **101A** and angle section **114A** or the like. It is alternatively contemplated herein that connector **130C** may preferably comprise a fixed connection therebetween first connector end **114A.3** of first connector section **114A.1** and first rod end **101A.1** of rod **101A.** Moreover, second connector section **114A.2** of angle section **114A** preferably includes a transition end or fitted end, such as second connector end **114A.5** configured to releasably connect second connector section **114A.2** of angle section **114A** thereto angle section **114B.** Preferably, second connector end **114A.5** includes receptacle **114A.7** and further includes first edge **114A.6** configured preferably to match, fit, (notched-slotted) and/or receive angle section **114B** to aid in the proper alignment of angle sections **114A/B** and to maintain rods **101A/B** in alignment or parallel with each other. It is contemplated herein that receptacle **114A.7** and/or first edge **114A.6** may be alternatively configured to accommodate and/or releasably connect second connector section **114A.2** of angle section **114A** thereto angle section **114B.**

Preferably, second connector section **114A.2** of angle section **114A** includes a pin, leg, support, or hanger, such as third peg **114A.8** configured approximately vertical, perpendicular or right angled to second connector section **114A.2** and extends above (third peg **114A.8.1**) and/or below (third peg **114A.8.2**) second connector section **114A.2.** Third peg **114A.8** is preferably inserted through conduit **114A.9** configured through second connector section **114A.2** or may have been formed as a part of second connector section **114A.2** or angle section **114A.** It is contemplated herein that third peg **114A.8** may alternatively have been positioned or formed as a part of first connector section **114A.1.** Furthermore, third peg **114A.8** may be configured with grommet or cover **114A.10** to releasably affix or hold third peg **114A.8** thereto apertures **340** formed tray top **320.**

It is contemplated herein that angle section **114A** may be formed or molded as one piece including first connector section **114A.1,** second connector section **114A.2,** and third peg **114A.8.**

Preferably angle section **114B** includes first connector section **114B.1** configured approximately perpendicular, curved, or right angled to second connector section **114B.2** and both are preferably adjoined to one another at a common intersection **114B.4.** First connector section **114B.1** preferably includes a transition end or tapered end, such as first connector end **114B.3** configured to releasably and slidably connect first rod end **101B.1** of rod **101B** thereto first connector section **114B.1** of angle section **114B.** It is recognized herein that connector **130D** may be an adhesive, compression fitting, weld, mechanical connector, single molded component of rod **101B** and angle section **114B** or the like. It is alternatively contemplated herein that connector **130D** may preferably comprise a fixed connection therebetween such as first connector end **114B.3** of first connector section **114B.1** and first rod end **101B.1** of rod **101B.** Moreover, second connector section **114B.2** of angle section **114B** preferably includes a transition end or fitted end, such as second connector end **114B.5** configured to releasably connect second connector section **114B.2** of angle section **114B** thereto angle section **114A.** Preferably, second connector end **114B.5** includes protrusion **114B.7** and further includes first edge **114B.6** configured preferably to match, fit, (notched-slotted) first edge **14A.6** and/or insert therein receptacle **114A.7** of angle section **104A** to aid in the proper alignment of angle sections **114A/B** and to maintain rods **101A/B** in alignment or parallel with each other. It is contemplated herein that protrusion **114B.7** and/or first edge **114B.6** may be alternatively configured to accommodate and/or releasably connect second connector section **114B.2** of angle section **114B** thereto angle section **114A.**

Preferably, second connector section **114B.2** of angle section **114B** includes a pin, leg, support, or hanger, such as fourth peg **114B.8** configured approximately vertical, perpendicular or right angled to second connector section **114B.2** and extends above (fourth peg **114B.8.1**) and/or below (fourth peg **114B.8.2**) second connector section **104B.2.** Fourth peg **114B.8** is preferably inserted through conduit **114B.9** configured through second connector section **114B.2** or may have been formed as a part of second connector section **114B.2** or angle section **114B.** It is contemplated herein that fourth peg **114B.8** may alternatively have been positioned or formed as a part of first connector section **114B.1.** Furthermore, fourth peg **114A.8** may be configured with grommet or cover **114B.10** to releasably affix or hold fourth peg **114A.8** thereto apertures **340** formed tray top **320.**

It is contemplated herein that angle section **114B** may be formed or molded as one piece including first connector section **114B.1,** second connector section **114B.2,** and fourth peg **114B.8.**

It is contemplated herein that pegs **104A.8, 104B.8, 114A.8,** and **114B.8** may be removeable or slid in conduit **104A.9, 104B.9, 114A.9,** and **114B.9,** respectively, to enable stringer **100A** to lie flat on a surface or to assist with stacking, sorting, inspecting, and counting of surgical instruments I, and additionally when positioning on a sorting table for a technician to inspect, count, and sort.

Stringer **100A** further includes two or more connectors **130A/B/C/D/E/F** for extension and retraction of rods **101A/B.** Preferably, connector **130A** is configured as a linear adjustment or latch and release connector operational in the x axis **X** and positioned between angle sections **104A** and **104B.** Preferably, second connector section **104A.2** of angle section **104A** and second connector section **104B.2** of angle section **104B are** configured as conduits with hollow passageway therethrough for insertion therein of a fastener, clasp, or latch mechanism, such as quick release pin **105A1/A2.**

Preferably, quick release pin **105A1/A2** includes main body tube **105A,** a bias mechanism, such as spring **105B,** a rod, or pin, such as spindle **105C,** and one or more balls **105D.** Main body tube **105A** preferably includes extension tube **105A.2** and alignment tube **105A.1** having a conduit or passageway **105A.4** therethrough with first access opening **105A.3** on one end of alignment tube **105A.1** and second access opening **105A.5** on one end of extension tube **105A.2** of body tube **105A.** Preferably one or more small holes **105A.5** are drilled or formed in side wall **105A.6** of extension tube **105A.2,** more specifically near one end or tip of extension tube **105A.2.** One or more balls **105D** are preferably positioned therein one or more small holes **105A.5,** wherein one or more small holes **105A.5** are preferably configured to maintain one or more balls **105D** in position to protrude through side wall **105A.6** of extension tube **105A.2** but not allow one or more balls **105D** to pass all the way through one or more small holes **105A.5** and fall out. Spindle **105C** preferably includes button **105C.1,** spring support **105C.2,** and spindle rod **105C.4.** Preferably, spindle rod **105C.4** is passed through spring **105B,** inserted in access opening **105A.3,** further into passageway **105A.4,** and further configured to contact or place pressure/force on the backside of one or more balls **105D** to hold one or more balls **105D** in one or more small holes **105A.5** of extension tube **105A.2.** It is contemplated herein that spindle rod **105C.2** may contain a small section that is machined down to a smaller diameter or wedge, such as machined section **105C.3,** where spindle rod **105C.2** contacts one or more balls **105D** in the release position. Moreover, spindle rod **105C.4** includes first spindle end **105C.3,** second spindle end **105C.6,** and machined section **105C.5** of spindle rod **105C.2.**

In use, when button **105C.1** on the end of spindle rod **105C.2** is pressed, such action compresses spring **105B** between button **105C.1** and main body tube **105A,** wherein spindle rod **105C.2** moves inward into passageway **105A.4** and enables one or more balls **105D** to retract into extension tube **105A.2** from their seated positions into machined section **105C.5** of spindle rod **105C.2** (retracted position). In the retracted position, one or more balls **105D** are preferably flush or slightly below the outside surface of extension tube **105A.2.** When the button is released spring **105B** provides pressure/force to pull spindle rod **105C.2** out of main body tube **105A** and force one or more balls **105D** to extend out of extension tube **105A.2** (extended position). In the extended position, one or more balls **105D** are preferably in contact with receptacle **104A.7** of second connector section **104A.2** of angle section **104A** releasably affixing angle section **104A** to angle section **104B.** It is contemplated herein that receptacle **104A.7** is preferably configured to receive extension tube **105A.2.** In the extended and retracted position, one or more balls **105D** of release pin **105A** are configured to provide latch and release capability in the x axis **X** between angle sections **104A** and **104B** via connector **130A.** It is contemplated herein that receptacle **104A.7** of second connector section **104A.2** may include one or more bored diameters to accommodate extension tube **105A.2** in its extended and retracted position to provide latch and release capability in the x axis **X** between angle sections **104A** and **104B.** It is further contemplated herein that receptacle **104A.7** of second connector section **104A.2** may include interior dimples or other configurations to catch one or more balls **105D** when in their extended position to assist with latch capability in the x axis **X** between angle sections **104A** and **104B.** It is still further contemplated herein that other latch and release or the like mechanisms may be utilized herein.

Preferably, connector **130B** (like connector **130C**) is configured as a linear adjustment or latch and release connector operational in the x axis **X** and positioned between angle sections **114A** and **114B.** Preferably, second connector section **114A.2** of angle section **114A** and second connector section **114B.2** of angle section **114B** are configured as conduits with hollow passageway therethrough for insertion therein of a fastener, clasp, or latch mechanism, such as quick release pin **105A2.** It is contemplated herein that quick release pin **105A** may be utilized to provide latch and release capability in the x axis **X** between angle sections **114A** and **114B.**

It is recognized herein that connector **130A** and connector **130B** enable travel, adjustment, extension, latch, release, or retraction between angle sections **104A** and **104B** and angle sections **114A** and **114B.**

Preferably connector section **130C** is positioned between rod **101A** and angle sections **114A,** connector section **130D** is positioned between rod **101B** and angle sections **114B,** connector section **130E** is positioned between rod **101B** and angle sections **104B,** and connector section **130F** is positioned between rod **101A** and angle sections **104A.** For assembly, of connector section **130C** first rod end **101A.1** of first rod section **101A** is preferably inserted in first connector end **114A.3** configured to releasably and slidably connect first rod end **101A.1** of rod **101A** thereto first connector section **114A.1** of angle section **114A.** For assembly, of connector section **130D** first rod end **101B.1** of first rod section **101B** is preferably inserted in first connector end **114B.3** configured to releasably and slidably connect first rod end **101B.1** of rod **101B** thereto first connector section **114B.1** of angle section **114B.** For assembly, of connector section **130E** second rod end **101B.2** of first rod section **101B** is preferably inserted in first connector end **104B.3** configured to releasably and slidably connect second rod end **101B.2** of rod **101B** thereto first connector section **104B.1** of angle section **104B.** For assembly, of connector section **130F** second rod end **101A.2** of first rod section **101A** is preferably inserted in first connector end **104A.3** configured to releasably and slidably connect second rod end **101A.2** of rod **101A** thereto first connector section **104A.1** of angle section **104A.** It is contemplated herein that connector section **130C/D/E/F** are preferably configured to enable travel along y axis **Y,** adjustment, positioning, or spacing therebtween angle sections **104A** and **114A,** and angle sections **104B** and **114B.** It is contemplated herein that connector section **130C/D/E/F** may be affixed to angle section **114A, 114B, 104B,** or **104A** utilizing a weld, adhesive, mechanical connector, compression fitting or the like.

It is contemplated herein that stringer **100A** may include a single axis of adjustment or telescope, such as along the x axis **X** or y axis **Y** or alternatively a multi axis adjustment or telescope, such as along the x axis **X** and y axis **Y.**

It is further contemplated herein that connector **130A/B/C/D/E/F** of stringer **100A** may include inner rod that is slidably longitudinally received within a hollow outer rod and that each end of a rod may be interchanged from inner to outer.

It is still further contemplated herein that connector **130A/B/C/D/E/F** of stringer **100A** may be interchanged.

It is still further contemplated herein that connector **130A/B/C/D/E/F** of stringer **100A** may include one or more expand and contract sections or slidable sections to enable x axis **X** and/or y axis **Y** parallel adjustment or spacing of the rods that form stringer **100A.**

Referring now to **FIG. 4****.****1** an exemplary surgical instrument **I**, such as a hemostat having moveable shank **S1** and shank **S2,** which preferably includes a pair of opposing first jaw member **J1** and second jaw members **J2,** jaw members, wherein at least one of the jaw members is movable relative to the other. Such jaw members may be pivotable about pivot point **P,** within box lock **B,** between first open position **O** in which the jaw members are disposed in a spaced relation relative to one another, and second closed position **C** in which the jaw members are configured to grasp or clamp something therebetween, such as tissue, or the like. Moreover, opposing first jaw member **J1** and second jaw members **J2** may be locked or held in position by a lock such as ratchet lock **L** preferably positioned on an extension stem or support member such as shank **S1** and shank **S2** between the two ring handles **R.** In use, for example a user positions their thumb in first ring handle **R1** and their index or middle finger within second ring handle **R2.** By a user opening and closing their thumb and index or middle finger this causes first ring handle **R1** and second ring handle **R2** to move apart (first open position **O**) and together (second closed position **C**), accordingly. The movements of first ring handle **R1** and second ring handle **R2** causes shank **S1** and shank **S2** to pivot about pivot point **P** which results in first jaw member **J1** and second jaw members **J2** to likewise pivot about pivot point **P** between first open position **O** and second closed position **C.**

Further, surgical instruments **I** may include hemostats, forceps, clamps, scalpels, scissors, picks, retractors, hooks, clips, pliers, punches, curettes, specula and the like, which are generally of high precision and intricate construction and come in a variety of types, shapes and sizes, all of which may be used during a particular surgical procedure. A variety of surgical tools exist for each category of instruments. For example, considering only forceps, they come straight, left curved, right curved, serrated, cupped, etc. In addition, a range of medical, dental and veterinary tools have been developed for each discipline, such as in medicine sub-categories of surgical procedures include anesthesia, cardio, dermatology, ear nose & throat, hand (specific limbs), facial, ob/gyn, orthopedic and the like each discipline having a variety of specialty and common surgical instruments. Over the years, surgical procedures along with the type and quantity of surgical instruments used in a given procedure have become predominantly standardized. Many of the above-mentioned instruments are scissor action instruments, having a pair of ring handles **R** that are connected through a pivot **P** to working moveable members shank **S1** and shank **S2.** Preferably, lever, extension or support members such as shank **S** typically include ring handles **R** at the ends thereof to facilitate the opening and closing of shank **S1** and shank **S2** connected to first jaw member **J1** and second jaw members **J2.**

Referring now to **FIG. 4****.****2** an exemplary embodiment of a plurality of surgical instruments **I** strung together by stringer **100A.** Preferably rod **101A** and rod **101B** of stringer **100A** are positioned through first ring handle **R1** and second ring handle **R2** of one or more surgical instruments **I** to group surgical instruments **I** in sequential order along y axis **Y.** Moreover, one exemplary function of the extension and retraction capability of connector **130C/F** (shown in **FIG. 3****)**and **130D/E** is to enable stringer **100A** to accommodate additional (expansion) or fewer (retraction) numbers of surgical instruments **I** along y axis **Y.** Such extension and retraction maintains a tight y axis **Y** fit around ring handles **R** of the group surgical instruments **I** and holds the group surgical instruments **I** in an erect position. Such erect position, space between instruments, and organization of the group surgical instruments **I** enables shortened time to identify, clean (whether pre-wash, pressure washing or the like), sort, count, group, and sterilize surgical instrument **I**. Moreover, such erect position, space between instruments, and organization of the group surgical instruments I reduces instrument damage when transporting, stacking, sorting, inspecting, and counting, and additionally when positioning on a sorting table for a technician to inspect, count, and sort. Still further, such extension and retraction of connector **130C/F** (shown in **FIG. 3****)** and **130D/E** enables stringer **100A** to adjust and accommodate a variety of sizes of surgical instruments **I1/12** and numbers or groups of surgical instruments **I**. It is contemplated herein that first peg **104A.8,** second peg **104B.8,** third peg **114A.8,** and fourth peg **114B.8** may be utilized to raise first ring handle **R1** and second ring handle **R2** of one or more surgical instruments **I** above a surface or plug or insert into a holed surface.

Procedurally the surgical instruments **I** should be strung post-surgery when in their aligned position on the surgical instrument roll by feeding stringer **100A** through first ring handle **R1** and second ring handle **R2.** Thereafter the surgical instruments **I** are preferably handled as a group of surgical instruments **I** bound together by stringer **100A** through the multi-step sorting, identifying, grouping, cleaning and sterilization process. Such bundling of surgical instruments **I** preferably helps to protect surgical instruments **I** from damage when transported, stacked one on the other as well as when the surgical instruments are emptied out on a sorting table (no longer required) for a technician to inspect, count, and sort. Such handling may scratch, bend and may even break the surgical instruments resulting in increased cost to replace such instruments, which are often delicate and expensive. Moreover, such damage to the surgical instruments reduces the life expectancy of the surgical instruments resulting in increased medical costs to replace the surgical instruments. Furthermore, if such damaged surgical instruments are accidentally returned to the operating room, such surgical procedures may be delayed or cancelled due to non-functioning surgical instruments causing lost revenue for the surgery center and an upset surgical team and patients in queue.

Moreover, one exemplary function of the release and latch capability of connectors **130A** and **130B** (shown in **FIG. 3****)** is to enable stringer **100A** to accommodate surgical instruments **I** in an open position **O** and upright. Such release and latch of connector **130A** and **130B** (shown in **FIG. 3****)** maintains x axis **X** spacing between first ring handle **R1** and second ring handle **R2** of the group surgical instruments **I**. Such movement of first ring handle **R1** and second ring handle **R2** causes shank **S1** and shank **S2** to pivot about pivot point **P** which results in first jaw member **J1** and second jaw members **J2** to likewise pivot about pivot point **P.** Preferably stringer **100A** holds the group surgical instruments **I** in an erect position with opposing first jaw member **J1** and second jaw members **J2** held in open position **O**. Such open position **O**, spacing between instruments, and organization of the group surgical instruments **I** enables more thorough cleaning and sterilization of surgical instruments **I**. In addition, maintaining such open position **O** between first jaw member **J1** and second jaw members **J2** preferably reduces the time to sort, identify, inspect, group, clean and sterilize the group surgical instruments **I**. Moreover, such open position **O** of the plurality of surgical instruments **I** strung together by stringer **100A** reduces the number of sharps being sent to central sterile and the reduction of accidents associated with needle/sharps injuries retained in a surgical instrument **I** or hidden in an un organized cluster of surgical instruments **I**. Such erect position, spacing between instruments, and organization of the group surgical instruments **I** enables shortened time to identify, clean (whether pre-wash, pressure washing or the like), sort, count, group, and sterilize surgical instrument **I**. Still further, such release and latch of connector **130A** and **130B** (shown **in** **FIG. 3****)** enables stringer **100A** to adjust and accommodate a variety of sizes of surgical instruments **I** and groups of surgical instruments **I**.

Referring now to **FIG. 5****.****1** there is illustrated a perspective view of an example embodiment box like tray bottom and lid surgical instrument support tray system **10** with exemplary adjustable stringer **100** position or affixed thereon. Preferably, surgical instrument support tray system **10** includes a container or basket and lid, such as tray bottom **220** and tray top **320.** Tray bottom **220** and tray top **320** are preferably formed of a suitable material, stainless steel, aluminum, metal, metal alloys, shape memory alloys, carbon fibers, ceramic, and includes chrome or other plated metals or coated metal and anodizing or the like, capable of providing structure to tray bottom **220** and tray top **320.** Preferably, the material includes other suitable characteristics, such as durability, rigidity, stain-resistance, bacteria-resistant, light weight, chemical inertness, oxidation resistance, ease of workability, color coding, or other beneficial characteristic understood by one skilled in the art.

Referring now to **FIG. 5****.****2****.****1** there is illustrated a perspective view of an example embodiment tray bottom **220.** Preferably, tray bottom **220** may be configured as a basket-like housing or enclosure in which surgical instruments **I** may be positioned, sterilized, transported and stored for later use. Tray bottom **220** comprises four generally perpendicular, upwardly projecting, continuous planar member(s), surface(s) or side(s) such as such as side walls **222, 223, 224, 225** and bottom **226** arranged preferably as a rectangle or square and having an open top box. Side walls **222-225** preferably define a generally open top having an upper perimeter **227.**

It is contemplated herein that one or more sides walls **222, 223, 224, 225** and bottom **226** may be formed from a wire grid construction.

It is further contemplated herein that tray bottom **220** may include a variety of shapes and sizes to accommodate a variety of surgical instruments **I**.

Referring again to **FIG. 5****.****2****.****1**, preferably side walls **222, 223, 224, 225** and bottom **226** may be formed or configured with a plurality or set of holes such as apertures **240** to enable steam or other sterilant to flow through apertures **240** formed in side walls **222, 223, 224, 225** and bottom **226** and permeate the entire interior of tray bottom **220.** Furthermore, such sterilant may pass up, under and over the surgical instruments **I** positioned in tray bottom **220,** thus ensuring effective sterilization.

Preferably, tray bottom **220** includes two or more wire handles such as handle wire **230** configured generally in a u-shape. Handle wire **230** is preferably positioned approximate an interior side of one of side walls **222, 223, 224,** or **225** (preferably **223** and **225**) and removably affixed thereto and configured to enable swivel movement of handle wire **230** about a plate such as handle retainer plate **232.** At least two handle retainer plates **232** are preferably affixed to side walls **223** and **225** by an attachment device such as machined screw **234** positioned through machined apertures **236** configured in side walls **223** and **225.** Handle wires **230** are preferably affixed to side walls **223** and **225** to facilitate the lifting, carrying and positioning of tray bottom **220.**

Furthermore, any of side walls **222, 223, 224, 225** and bottom **226** may include an identifying legend such as indicia **231** to enable identification of the surgical instruments **I** contained therein or the surgical procedure identified with the set or group of surgical instruments **I** contained within tray bottom **220,** surgical kit identification, dates of sterilization, dates of expiration, model number, serial number, ownership and the like.

Referring now to **FIG. 5****.****2.2** there is illustrated a perspective view of an example embodiment surface, lid or cover, such as tray top **320** of the surgical instrument support tray system **10.** Preferably, tray top **320** may be configured as a box top, cover, closure or lid for tray bottom **220** in which surgical instruments **I** may be positioned, removably affixed thereto, sterilized, transported and stored for later use. Tray top **320** comprises four generally perpendicular, downwardly projecting, continuous, planar member(s), surface(s) or side(s) arranged preferably as a rectangle or square, such as side walls **322-325.** Side walls **322-325** preferably define a top having perimeter **227** configured to engage, surround, or friction fit side walls **222-225** of tray bottom **220** to form an enclosure in which surgical instruments **I** may be positioned, removably affixed thereto, sterilized, transported and stored for later use. It is contemplated herein that tray bottom **220** and tray top **320** may include a latch mechanism to secure tray bottom **220** and tray top **320** to one another. Furthermore, any of side walls **322, 323, 324, 325** and/or top **326** may be configured as a support member for surgical instruments **I**, such as top **326.**

Likewise, side walls **322, 323, 324, 325** and top **326** may be formed or configured with a plurality or set of holes such as apertures **340** to enable steam or other sterilant to flow through apertures **340** formed in side walls **322, 323, 324, 325** and top **326** and permeate the entire interior of tray top **320** and into the interior of tray bottom **220.** Furthermore, such sterilant may pass down through tray top **320** and over the surgical instruments **I** positioned in tray bottom **220,** thus ensuring effective sterilization.

It is contemplated herein that tray bottom **220** and tray top **320** may be configured in shapes other than square and rectangle provided such configuration enables sterilization, transport and storage for later use of surgical instruments **I**.

Referring again to **FIG. 5****.****2.2**, preferably top **326** of tray top **320** includes regularly spaced columns or rows of a plurality or set of furrows, trenches, channel or troughs, such as valleys **650** formed therein top **326.** Valleys **650,** such as first valley **651** and second valley **652,** are formed as a pair of columns in top **326** of tray top **320,** and are preferably configured as a furrows, trenches, channel or troughs shape positioned parallel to side walls **323** and **325,** into which portions of surgical instrument **I**, preferably ring handles **R** may be held or positioned. Preferably, ring handles **R** may be positioned and/or held in a desired and evenly spaced position therein valleys **650** preventing surgical instrument **I** from coming into contact with one another, intermingling, and enabling easy identification, cleaning, sorting, counting, and grouping of surgical instruments **I**. Valleys **650** are preferably formed in sets of two to accommodate stringer **100** and a group of ring handles **R** of surgical instruments **I** bound together by stringer **100.** Moreover, one or more pins, plugs, screws, rivets, bolts, clips or other fastener, such as stringer attachment device **700** may be utilized to affix stringer **100** and/or a group of surgical instruments **I** bound together by stringer **100** to tray top **320.** It is contemplated herein that stringer attachment device **700,** more specifically first attachment device **702** and second attachment device **704,** may affix stringer **100** or a group of surgical instruments **I** bound together by stringer **100** to one or more apertures **340** in side walls **322, 323, 324, 325** and/or top **326,** especially apertures **340** adjacent perimeter **227.**

Referring now to **FIG. 5****.****3****.****1** there is illustrated a top view of an example embodiment lid or cover, such as tray top **320** of the surgical instrument support tray system **10.** Preferably top **326** of tray top **320** includes regularly spaced **S** columns or rows of a plurality of valleys **650,** such as first valley **651,** second valley **652,** third valley **653** and the like, formed therein top **320.** Valleys **650** are preferably spaced **S** distance apart to accommodate, cup and support ring handles **R** of surgical instrument **I** therein. In addition, valleys **650** are preferably spaced **S** distance apart to maintain surgical instrument **I** in open position **O**, shown in **FIG. 4****.** Preferably top **326,** includes valleys **650,** as a pair, such as first valley **651** and second valley **652,** are formed as a pair of columns, and are preferably configured as a furrows, trenches, channel or troughs shape positioned parallel to side walls **323** and **325,** into which portions of surgical instrument **I**, such as ring handles **R** may be positioned or held. Preferably, ring handles **R** may be positioned and/or held in a desired and evenly spaced position therein valleys **650** in top **326** preventing surgical instrument **I** from coming into contact with one another, intermingling, and enabling easy identification, cleaning, sorting, counting, and grouping of surgical instruments **I**.

Referring now to **FIG. 5****.****3.3** there is illustrated a cross sectional side view along **AA** of tray top, such as tray top **320** of the surgical instrument support tray system **10.** Preferably top **326** shown from side wall **324,** includes valleys **650,** as a plurality of pairs, such as first valley **651** and second valley **652** (first pair) and third valley **653** and fourth valley **654** (second pair), each formed as a pair into which portions of surgical instrument **I**, preferably one or more sets of ring handles **R** may be positioned or held preventing surgical instrument **I** from coming into contact with one another, intermingling, and enabling easy identification, cleaning, sorting, counting, and grouping of surgical instruments **I**.

Referring now to **FIG. 5****.****3****.****2** there is illustrated a side cross sectional view of tray top **320** along line **A-A** showing cross sectional of surface **326** of the surgical instrument support tray system **10.** Preferably, valleys **650,** such as second valley **652,** third valley **653** and the like, formed therein top **320.** Preferably, valleys **650,** such as second valley **652** includes a curved, arced or bent surface, such as second contoured surface **662** and end edge **664,** and third valley **653** includes third contoured surface **663** and end edge **664.** Moreover, therebetween and on each side of valleys **650,** such as second valley **652** and third valley **653** there is shown cross sectional of surface **326.** Preferably contoured surfaces, such as second contoured surface **662** and third contoured surface **663** are formed in top **320** and spaced **S** distance apart to position or hold ring handles **R** in a desired and evenly spaced position when positioned therein valleys **650** to prevent surgical instrument **I** from coming into contact with one another, intermingling, and enabling easy identification, cleaning, sorting, counting, and grouping of surgical instruments **I**. It is contemplated herein that second contoured surface **662** and third contoured surface **663** may be configured or formed as polygon, curved or the like to conform to, position, or hold ring handles **R** of surgical instrument **I**.

### Manufacture

Preferably, tray bottom **220,** tray top **320** and contoured surface **662** and contoured surface **663** of valleys **650** are formed as follows: a turret machine is preferably utilized to punch all apertures **240** and **340** in a stock sheet of aluminum or stainless steel and to cut outside perimeter shape, such as perimeter **227.** Next, "brake press" (a large hydraulic press machine) is preferably used to press one or more valleys **650** into surface **326** of tray top **320.** Next, the brake press is preferably utilized to form side walls **322, 323, 324, 325** of tray top **320.** It is contemplated herein that large volumes of production of tray top **320** may be formed using automated processes known to one of ordinary skill in the art. For example, preferably utilizing a larger "die" shaped to press all apertures **240** and **340** in a stock sheet of aluminum or stainless steel and to cut outside perimeter shape, such as perimeter **227** in in one hit/press. Next, a similar press is preferably utilized to press one or more valleys **650** into surface **326** of tray top **320** and to form side walls **322, 323, 324, 325** of tray top **320** in one hit/press. It is contemplated herein that tray bottom **220** may be configured or formed with one or more valleys **650,** similar to tray top **320,** formed in bottom **226** of tray bottom **220,** to accommodate ring handles **R** may, which may be positioned and held in a desired and evenly spaced position preventing surgical instrument **I** from coming into contact with one another, intermingling, and enabling easy identification, cleaning, sorting, counting, and grouping of surgical instruments **I**. Referring again to **FIG 5****.****3****.****2** there is illustrated a side cross sectional view of tray bottom **220** showing cross sectional surface **326** of the surgical instrument support tray system **10.**

Referring now to **FIG. 5****.****4****.****1** and **5.4.2** there is illustrated a top and cross sectional view of an example embodiment tray bottom **220.** Preferably tray bottom **220** includes one or more fastener, hanger, peg, or arm, such as thumb screw **252** and **254,** more specifically thumb screws **252A, 252B, 252C,** and **252D** for insertion in apertures **242A, 242B, 242C,** and **242D** (shown in **FIG.5****.****2**) of side walls **222** and thumb screws **254A, 254B, 254C,** and **254D** for insertion in apertures **244A, 244B, 244C,** and **244D** (shown in **FIG.5****.****2**) of side walls **224.** Thumb screw **252** and **254** are preferably inserted in apertures **242A, 242B, 242C,** and **242D** (shown in **FIG.5****.****2**) in side walls **222** and in apertures **244A, 244B, 244C,** and **244D** (shown in **FIG.5****.****2**) in side walls **224,** respectively, and utilized to support an inner tray, such as surgical instrument support tray system **10.1.** It is contemplated herein that surgical instrument support tray system **10.1** may be color coordinated and/or grouped within surgical instrument support tray system **10** for easy identification of assemblies for specific surgical specialties. Preferably, surgical instrument support tray system **10.1** may be configured or utilized to support specialty, random, non-ring handled surgical instruments or other surgical instruments. It is contemplated that side walls **222** and **224** may include one or more parallel first set of thumb screws **252A, 252B, 252C,** and **252D** and one or more parallel second set of thumb screws **254A, 254B, 254C,** and **254D** or the like to provide support and accommodate a variety of configured inner tray(s), such as surgical instrument support tray system **10.1.**

Referring again to **FIG. 5.5****,** preferably top **326** of tray top **320** includes regularly spaced columns or rows of a plurality or set of furrows, trenches, channel or troughs, such as valleys **650** formed therein top **326.** Valleys **650,** such as first valley **651** and second valley **652,** are formed as a pair of columns in top **326** of tray top **320,** and are preferably configured as a furrows, trenches, channel or troughs shape positioned parallel to side walls **323** and **325,** into which portions of surgical instrument **I**, preferably ring handles **R** may be held or positioned. Preferably, ring handles **R** may be positioned and/or held in a desired and evenly spaced position therein valleys **650** preventing surgical instrument **I** from coming into contact with one another, intermingling, and enabling easy identification, cleaning, sorting, counting, and grouping of surgical instruments **I**. Valleys **650** are preferably formed in sets of two to accommodate stringer **100A** and a group of ring handles **R** of surgical instruments **I** bound together by stringer **100A.** Moreover, one or more pegs, such as that first peg **104A.8,** second peg **104B.8,** third peg **114A.8,** and fourth peg **114B.8** may be utilized to releasably connect or plug stringer **100A** and/or a group of surgical instruments **I** bound together by stringer **100A** to tray top **320.** It is contemplated herein that one or more pegs, more specifically first peg **104A.8,** second peg **104B.8,** third peg **114A.8,** and fourth peg **114B.8,** may releasably connect stringer **100A** or a group of surgical instruments **I** bound together by stringer **100A** to one or more apertures **340/340A** in side walls **322, 323, 324, 325** and/or top **326,** especially apertures **340** adjacent perimeter **227** of top **326.**

It is contemplated herein that cover or grommet **504A** and grommet **514A** may be inserted in one or more apertures **340/340A** and configured to releasably affix or hold one or more pegs, more specifically first peg **104A.8** of angle section **104A** and third peg **114A.8** of angle section **114A** to prevent angle section **104A** and angle section **114A (**stationary side of stringer **100A)** from dislodging, during use, from apertures **340/340A** in top **326.**

Referring now to **FIG. 6****.****1** there is illustrated a perspective view of an example embodiment of a surgical instrument support tray system **10** with exemplary adjustable stringers **100** shown adjusted in two positions affixed to tray top **320** of the surgical instrument support tray system **10.** Preferably, surgical instrument support tray system **10** includes tray bottom **220** and tray top **320,** and tray top **320** includes a plurality of regularly spaced valleys **650** formed therein top **326,** such as first valley **651,** second valley **652,** third valley **653,** fourth valley **654,** fifth valley **655,** sixth valley **656**, seventh valley **657,** and eighth valley **658.** Preferably, stringer **100A** is adjusted, positioned, or expand and contract along slidable coupler sections **130A/B/C/D** of stringer **100A** to enable x axis **X** and/or y axis **Y** parallel adjustment or spacing of the rods that form stringer **100A** to be positioned approximate first valley **651** and third valley **653** of tray top **320.** Alternatively, stringer **100B** is adjusted, positioned, or expand and contracted along slidable coupler sections **130A/B/C/D** of stringer **100B** to enable x axis **X** and/or y axis **Y** parallel adjustment or spacing of the rods that form stringer **100B** to be positioned approximate fifth valley **655** and sixth valley **656** of tray top **320.** It is contemplated herein that slidable coupler sections **130A/B/C/D** of stringer **100A/B** may be adjusted, retracted, or expand and contracted along slidable coupler sections **130A/B/C/D** of stringer **100A/B** to enable x axis **X** and/or y axis **Y** parallel adjustment, positioning, or spacing of the rods that form stringer **100A/B** to be positioned approximate a pair or a plurality of valleys **650** of tray top **320.**

It is contemplated herein that stringer **100A/B** may be color coordinated and/or grouped within inner tray, such as surgical instrument support tray system **10.1** and/or surgical instrument support tray system **10** for easy identification of assemblies for specific surgical specialties.

Referring now to **FIG. 6.1A** there is illustrated a perspective view of an example embodiment of tray top **320** with exemplary stringers **100A** shown adjusted in two positions affixed to tray top **320.** Preferably, tray top **320** includes a plurality of regularly spaced valleys **650** formed therein top **326,** such as first valley **651,** second valley **652,** third valley **653,** fourth valley **654,** fifth valley **655,** sixth valley **656**, seventh valley **657,** and eighth valley **658.** Preferably, stringer **100A1** is latched, adjusted, positioned, released, or expands and contracts along connector sections **130A/B/C/D/E/F** of stringer **100A** to enable x axis **X** and/or y axis **Y** parallel adjustment or spacing of the rods that form stringer **100A1** and to be positioned approximate first valley **651** and second valley **652** of tray top **320.** Alternatively, stringer **100A2** is latched, adjusted, positioned, released, or expands and contracts along connector sections **130A/B/C/D/E/F** of stringer **100A2** to enable x axis **X** and/or y axis **Y** parallel adjustment or spacing of the rods that form stringer **100A2** to be positioned approximate seventh valley **657** and eighth valley **658** of tray top **320.** It is contemplated herein that connector sections **130A/B/C/D/E/F** of stringer **100A1/A2** may be latched, released, adjusted or expand and contract along connector sections **130A/B/C/D/E/F** of stringer **100A1/A2** to enable x axis **X** and/or y axis **Y** parallel adjustment, positioning, or spacing of the rods that form stringer **100A1/A2** to be positioned approximate a pair or a plurality of valleys **650** of tray top **320.** It is contemplated herein that one or more pegs, more specifically first peg **104A.8,** second peg **104B.8,** third peg **114A.8,** and fourth peg **114B.8,** may be releasably positioned or inserted therein apertures **340/340A** to affix stringer **100A1/A2** or a group of surgical instruments **I** bound together by stringer **100A1/A2** to one or more apertures **340/340A** in side walls **322, 323, 324, 325** and/or top **326,** especially apertures **340/340A** adjacent perimeter **227** of top **326.**

It is contemplated herein that stringer **100A1/A2** may be color coordinated and/or grouped within inner tray, such as surgical instrument support tray system **10.1** and/or surgical instrument support tray system **10** for easy identification of assemblies for specific surgical specialties.

It is further contemplated herein that cover or grommet **504A** and grommet **514A** may be inserted in one or more apertures **340/340A** of top **326** and configured to releasably affix or hold one or more pegs, more specifically first peg **104A.8** of angle section **104A** and third peg **114A.8** of angle section **114A** of stringer **100A1** and 180 degree rotated first peg **104A.8** of angle section **104A** and third peg **114A.8** of angle section **114A** of stringer **100A2;** thus, to prevent angle section **104A** and angle section **114A** (stationary side of stringer **100A1/A2)** from dislodging, during use, from apertures **340/340A** in top **326.**

Referring now to **FIG. 6****.****2** there is illustrated a perspective view of an example embodiment of a surgical instrument support tray system **10** with a plurality of surgical instrument sets **I** held in a vertical position by exemplary adjustable stringers **100A/B** affixed or positioned approximate to tray top 320 of surgical instrument support tray system **10.** Preferably, surgical instrument support tray system **10** includes tray bottom **220** and tray top **320,** and tray top **320** includes a plurality of regularly spaced valleys **650** formed therein top **326.** For example, stringer **100A** positions and holds ring handle **R1** in valley **651** and positions and holds ring handle **R2** in valley **653** of tray top **320** for surgical instruments **IA.** Such positioning of surgical instruments **IA** preferably maintains opposing first jaw member **J1** and second jaw members **J2** in open position **O** for cleaning and sterilization purposes. Furthermore, such positioning of surgical instruments **IA** preferably maintains the instruments in a desired and evenly spaced position preventing them from coming into contact with one another, intermingling, and enabling easy identification, cleaning, sorting, counting, and grouping of surgical instruments **IA.**

As an alternative example, stringer **100B** positions and holds ring handle **R1** in valley **655** and positions and holds ring handle **R2** in valley **656** of tray top **320** for surgical instruments **IB.** Such positioning of surgical instruments **IB** preferably maintains opposing first jaw member **J1** and second jaw members **J2** of surgical instruments **IB** in open position **O** for cleaning and sterilization purposes. Furthermore, such positioning of surgical instruments **IB** preferably maintains the instruments in a desired and evenly spaced position preventing them from coming into contact with one another, intermingling, and enabling easy identification, cleaning, sorting, counting, and grouping of surgical instruments **IB.**

Moreover, one or more stringer attachment device **700** may be utilized to affix stringer **100A/B** and/or a group of surgical instruments **I** bound together by stringer **100A/B** to tray top **320.** It is contemplated herein that stringer attachment devices **700,** more specifically first stringer attachment devices **700A** and second stringer attachment devices **700B,** may affix stringer **100A/100B** or a group of surgical instruments **I** bound together by stringer **100A/B** to one or more apertures **340** in top **326,** especially apertures **340** adjacent perimeter **227.**

Referring now to **FIG. 6.2A** there is illustrated a perspective view of an example embodiment of tray top **320** with exemplary stringers **100A1/A2** shown adjusted in two positions affixed to tray top **320** with a plurality of surgical instrument sets **I** held in a proximately vertical position by exemplary stringers **100A1/A2** affixed, connected, or positioned approximate to tray top **320.** Preferably, tray top **320** includes a plurality of regularly spaced valleys **650** formed therein top **326.** For example, stringer **100A1** positions and holds ring handle **R1** in first valley **651** and positions and holds ring handle **R2** in second valley **652** of tray top **320** for surgical instruments **IA.** Such positioning of surgical instruments **IA** preferably maintains opposing first jaw member **J1** and second jaw members **J2** in open position **O** for cleaning and sterilization purposes. Furthermore, such positioning of surgical instruments **IA** preferably maintains the instruments in a desired and evenly spaced position preventing them from coming into contact with one another, intermingling, and enabling easy identification, cleaning, sorting, counting, and grouping of surgical instruments **IA.**

As an alternative example, stringer **100A2** positions and holds ring handle **R1** in valley **657** and positions and holds ring handle **R2** in valley **658** of tray top **320** for surgical instruments **IB.** Such positioning of surgical instruments **IB** preferably maintains opposing first jaw member **J1** and second jaw members **J2** of surgical instruments **IB** in open position **O** for cleaning and sterilization purposes. Furthermore, such positioning of surgical instruments **IB** preferably maintains the instruments in a desired and evenly spaced position preventing them from coming into contact with one another, intermingling, and enabling easy identification, cleaning, sorting, counting, and grouping of surgical instruments **IB.**

Moreover, one or more pegs, more specifically first peg **104A.8,** second peg **104B.8,** third peg **114A.8,** and fourth peg **114B.8,** may be positioned, connected, or inserted in apertures **340/340A** to releasably affix stringer **100A1/A2** or a group of surgical instruments **IA/IB** bound together by stringer **100A1/A2** to one or more apertures **340/340A** in top **326,** especially apertures **340/340A** adjacent perimeter **227.**

Referring now to **FIG. 6.3A** there is illustrated a perspective view of an example embodiment of tray top **320** with exemplary stringer **100A** shown unlatched in two positions, such as connector sections **130A/B** and stringer **100A** is further affixed to tray top **320** with a plurality of surgical instrument sets **I** held in a proximately vertical position by exemplary stringers **100A** affixed or positioned approximate to tray top **320.** Preferably, stringer **100A** is unlatched in two positions, such as connector sections **130A/B** to enable assembly **A1,** such as rod **101A,** and angle sections **104A/114A** to be releasably positioned, affixed or inserted by pegs **104A.8/114A.8** to apertures **340/340A** of tray top **320** and to be positioned in regularly spaced valleys **650,** such as valley **651,** and assembly **A2,** such as rod **101B,** and angle sections **104B/114B** to be releasably positioned, affixed or inserted by pegs **104B.8/114B.8** to apertures **340/340A** of tray top **320** and to be positioned in any regularly spaced valleys **650,** such as valley **654.** Such configuration enables longer shanked surgical instrument sets **I**, such as shank **S1** and shank **S2,** to be positioned with opposing first jaw member **J1** and second jaw members **J2** of surgical instruments **I** in wide open position **O** for better cleaning and sterilization purposes and to enable easy identification, pressure washing, sorting, counting, and grouping of surgical instruments **I**. It is contemplated herein that assembly **A1,** such as rod **101A,** and angle sections **104A/114A** and/or assembly **A2,** such as rod **101B,** and angle sections **104B/114B;** may be affixed by receptacle **104A.7** of second connector end **104A.5** of angle sections **104A;** receptacle **114A.7** of second connector end **114A.5** of angle sections **114A;** protrusion **104B.7** of second connector end **104B.5** of angle sections **104B;** and/or protrusion **114B.7** of second connector end **114B.5** of angle sections **114B;** respectively, configured to preferably match, fit, affix, insert, or latch apertures **340/340A** of tray top **320,** especially apertures **340/340A** adjacent perimeter **227.** Such configuration enables longer shanked surgical instrument sets **I**, such as shank **S1** and shank **S2,** to be positioned with opposing first jaw member **J1** and second jaw members **J2** of surgical instruments **I** in wide open position **O** for better cleaning and sterilization purposes and to enable easy identification, pressure washing, sorting, counting, and grouping of surgical instruments **I**.

It is further contemplated that receptacle **104A.7** and receptacle **114A.7** may releasably affix to a pin or peg (similar to peg **104A.8**) formed in tray top **320.**

It is further contemplated herein that assembly **A1** may be positioned in any of regularly spaced valleys **650** and assembly **A2** may be positioned in any other regularly spaced valleys **650.**

Referring now to **FIG. 6.4A** there is illustrated a perspective view of an example embodiment of tray top **320** with exemplary stringer **100A** shown unlatched in two positions, such as connector sections **130A/B** (shown in **FIG. 3****)**and stringer **100A** is further releasably affixed to tray top **320** with a plurality of surgical instrument sets **IA/B** held in a proximately vertical position by exemplary stringers **100A** releasably affixed or positioned approximate to tray top **320.** Preferably, stringer **100A** is unlatched in two or more positions, such as connector sections **130A/B** (shown in **FIG. 3****)** and at least slidably adjusted by connector sections **130C/F** (shown in **FIG. 3****)** of stringer **100A** to enable assembly **A1,** such as rod **101A,** and angle sections **104A/114A** (shown in **FIG. 6.3A)** to be releasably affixed by pegs **104A.8/114A.8** (shown in **FIG. 3****)** to apertures **340/340A** of tray top **320** and to be positioned in regularly spaced valleys **650,** such as valley **651,** and at least slidably adjusted by connector sections **130D/E** (shown in **FIG. 3****)** of stringer **100A** to enable assembly **A2,** such as rod **101B,** and angle sections **104B/114B** (shown in **FIG. 3****)** to be releasably affixed by pegs **104B.8/114B.8** (shown in **FIG. 3****)** to apertures **340/340A** of tray top **320** and to be positioned in regularly spaced valleys **650,** such as across valleys **653** and **654.**

Such configuration enables shorted shanked surgical instrument sets **I**, such as shank **S1** and shank **S2** of surgical instruments **IA** and longer shanked surgical instrument sets **IB,** such as shank **S1** and shank **S2** of surgical instruments **IB,** to be to be grouped on a common stringer **100A** and positioned with opposing first jaw member **J1** and second jaw members **J2** of surgical instruments **IA/B** in wide open position **O** for better cleaning and sterilization purposes and to enable easy identification, pressure washing, sorting, counting, and grouping of surgical instruments **IA/B.** It is further contemplated herein that assembly **A1,** such as rod **101A**, and angle sections **104A/114A** and/or assembly **A2,** such as rod **101B,** and angle sections **104B/114B** (shown in **FIG. 6.3A**) may be affixed by receptacle **104A.7** of second connector end **104A.5** of angle sections **104A;** receptacle **114A.7** of second connector end **114A.5** of angle sections **114A;** protrusion **104B.7** of second connector end **104B.5** of angle sections **104B;** and/or protrusion **114B.7** of second connector end **114B.5** of angle sections **114B;** (shown in **FIG. 3****)** respectively, configured preferably to match, fit, affix, insert, or latch any of apertures **340/340A** of tray top **320,** whether apertures **340/340A** are adjacent perimeter **227** or positioned interior to perimeter **227.** Such configuration enables shorter shanked surgical instrument sets **I**, such as shank **S1** and shank **S2** of surgical instruments **IA** and longer shanked surgical instrument sets **IB,** such as shank **S1** and shank **S2** of surgical instruments **IB** to be positioned with opposing first jaw member **J1** and second jaw members **J2** of surgical instruments **IA/B** in wide open position **O** for better cleaning and sterilization purposes and to enable easy identification, pressure washing, sorting, counting, and grouping of surgical instruments **IA/B.** Moreover, stringer **100A** accommodates shorter shanked surgical instrument sets **IA** and longer shanked surgical instrument sets **IB,** on one stringer **100** in wide open position **O** for better cleaning and sterilization purposes and to enable easy identification, pressure washing, sorting, counting, and grouping of surgical instruments **IA/B.**

Referring now to **FIG. 7****.****1** there is illustrated an exploded perspective view of the attachment device of **FIG. 5****.****2.2****.** Preferably, stringer attachment device **700** includes pan head bolt **780,** coil spring **760,** cylindrical spacer **740,** clip **720** and threaded end cap **710.** One or more stringer attachment devices **700** may be utilized to affix a portion of stringer **100** and/or a group of surgical instruments **I** bound together by stringer **100** to tray top **320.**

It is contemplated herein that stringer attachment device **700** includes other configurations of attachment devices known to one of ordinary skill in the art.

Referring now to **FIG. 7****.****2** there is illustrated a perspective view of an exemplary alternate stringer attachment device **801.** Referring now to **FIG. 7****.****2.2** there is illustrated a perspective view of an alternate stringer attachment device **801** that preferably includes releasably friction clip, snap-in clasp, or channel, such as trough **802** having first trough wall **804** and second trough wall **806,** base **807,** and pin, dowel, threaded bolts, screws, pine tree plugs or the like, such as pegs **808.** Referring now to **FIG. 7****.****2****.****3** there is illustrated a side view of an alternate stringer attachment device **801** having pegs **808,** base **807,** and first trough wall **804.** Referring now to **FIG. 7****.****2****.****1** there is illustrated a perspective view of one or more exemplary alternate stringer attachment device **801** in combination with stringer attachment device **700** in use. Preferably, alternate stringer attachment device **801** is positioned on top **326** of tray top **320** and aligned with apertures **340** in top **326,** especially apertures **340** adjacent perimeter **227.** Moreover, pegs **808** are preferably pushed through apertures **340** adjacent perimeter **227** and friction fit therein to top **326** or secured thereto top **326** by nuts or other attachment mechanism know to one of ordinary skill. One or more alternate stringer attachment device **801** may be utilized to affix or frictionally secure a portion of stringer **100** and/or a group of surgical instruments **I** bound together by stringer **100** to tray top **320.**

### Procedure

Referring again to **FIG. 7****.****2****.****1** there is illustrated either stringer attachment device **700** or alternate stringer attachment devices **801** affixed to tray top **320,** which may be additionally utilized to position and frictionally affix one end, side, rod or section of stringer **100,** such as slidable coupler section **130A,** of stringer **100** to tray top **320** while the other end, of stringer **100** is swung or hinged up or vertically positioned or placed with rods **102A** and **102B** perpendicular or adjacent to top **326** of tray top **320.** Moreover, slidable coupler sections **130C** and **130D** may be temporarily removed to enable stringing of ring handles **R** of surgical instrument **I** onto rods **102A** and **102B.** Once ring handles **R** of surgical instrument **I** have preferably been strung onto rods **102A** and **102B** slidable coupler sections **130C** and **130D** may be reattached to stringer **100** to group together surgical instruments **I** and stringer **100.** Preferably, stringer **100** is adjusted or expand and contract along connector **130A/B/C/D/E/F** of stringer **100** to position stringer **100** approximate valleys **650** and thereafter the other end, such as connector **130B** of stringer **100** is re-affixed to either stringer attachment device **700** or alternate stringer attachment devices **801,** as shown in **FIG. 6****.**

Referring to **FIG. 8** there is illustrated a flow diagram **800** of a method of organizing, sorting, identifying, grouping, counting, cleaning, pressure washing, sterilizing, and storing prior to surgical use with decreased damage to surgical instruments, and decreased assembly time for sterile surgical instrument sets utilizing, in step **805,** providing stringer **100/100A** and surgical instrument support tray system **10,** shown in **FIGs 1-7****.**

In block or step **810** and as described above in **FIGS 1-7****,** upon completion of surgery the operating personnel break down the surgical case and the surgical instruments **I** are sorted and stringer **100/A** rods, such as rods **101A/102A** and **101B/102B** may be inserted in ring handles **R** of surgical instruments **I** to group the set of surgical instruments **I** by stringing a row of ring handles **R** of surgical instruments **I** or by the procedure above for stringer attachment device **700** or attachment devices **800.** The grouped set of surgical instruments **I** may be positioned in valleys **650,** step **815** and **830,** by adjusting stringer **100/A** wherein the surgical instruments **I** may be identified, counted, sorted and positioned in an organized, parallel relationship in valleys **650** to form a set or group of surgical instruments **I**. While surgical instruments **I** have been retained, even spaced with open jaw by stringer **100/A** and surgical instrument support tray system **10** an assembler may quickly inspect, identify, sort, count, disengage any of the surgical instruments **I** for further inspection and operational testing, and grouping of surgical instruments **I** (inspecting step **835**). Thus, surgical instrument support tray system **10** reduces the time spent cleaning, sorting, counting, identifying and grouping surgical instruments **I,** extends the life expectancy of the surgical instruments, and enables thorough cleaning of the surgical instruments.

In block or step **820,** surgical instruments **I** may be gathered together on one end of stringer **100,** stringer **100** may be contracted or adjusted, and laid as a group of surgical instruments **I** on bottom **226** of tray bottom **220** and tray top **320** may be positioned on tray bottom **220** wherein the basket-like housing or enclosure maintains surgical instruments **I** in an organized and secure position for sterilization, transportation and storage for later use.

In block or step **830,** the grouped set of surgical instruments **I** may be removed from tray bottom **220** and the grouped set of surgical instruments **I** may be positioned in or across valleys **650** on tray top **320** by adjusting stringer **100** wherein the surgical instruments **I** may be retained in parallel or nonparallel, evenly spaced and an open jaw position by surgical instrument support tray system **10.** In such position, the grouped set of surgical instruments **I** may be sprayed and evenly coated with an enzymatic to begin breaking down post-surgery contaminants (pre-washing step **840**).

In block or step **832,** the grouped set of surgical instruments **I** may be positioned in or across valleys **650** on tray top **320** by adjusting stringer **100A** wherein the surgical instruments **I** may be retained in parallel or nonparallel, evenly spaced and an open jaw position by surgical instrument support tray system **10** by releasably affixing or inserting pegs **104A/B.8/114A/B.8** to or in apertures **340/340A** of tray top **320,** and configured to accommodate shorter shanked surgical instrument sets **IA** and longer shanked surgical instrument sets **IB,** on one stringer **100A** in wide open position **O** for better cleaning and sterilization purposes and to enable easy identification, pressure washing, sorting, counting, and grouping of surgical instruments **IA/B.**

In block or step **840,** the grouped surgical instruments **I** and stringer **100** may be pre-washed by submerging into a detergent pre-wash solution or sonification system. In addition, grouped surgical instruments **I** may be positioned in valleys **650** (step **830)** on tray top **320** by adjusting stringer **100** (step **820)** wherein the surgical instruments **I** may be scrubbed or pressure washed to remove any visible contaminants remaining post-surgery and inspected for completeness (pre-washing step **840**).

In block or step **850,** surgical instruments **I** may be gathered together on one end of stringer **100,** stringer **100** may be contracted or retracted (step **850**) with retained parallel spaced and open jaw surgical instruments **I,** and/or laid as a group of surgical instruments **I** on bottom **226** of tray bottom **220** and tray top **320** may be positioned on tray bottom **220** wherein the grouped surgical instruments **I** or basket-like housing or enclosure with surgical instruments **I** in an organized and secure position and open jaw surgical may be washed in a commercial washer/disinfector (step **845**). Since surgical instruments **I** are retained in parallel, evenly spaced and in an open jaw position this enables even access of detergents and uniform cleaning of surgical instruments **I** by a commercial washer/ disinfector.

In block or step **830,** the grouped set of surgical instruments **I** may be removed from tray bottom **220** and the grouped set of surgical instruments **I** may be positioned in or across valleys **650** on tray top **320** by adjusting stringer **100** wherein the surgical instruments **I** may be retained in parallel, evenly spaced and an open jaw position by surgical instrument support tray system **10.** While retained an assembler inspects, identifies, sorts and counts surgical instruments **I** (step **835).** Since surgical instruments **I** have been previously retained in parallel, even spaced with open jaw on surgical instrument support tray system **10** an assembler may quickly inspect, identify, sort, count, disengage any of the surgical instruments **I** for further inspection and operational testing, and finally grouping of surgical instruments **I**. Moreover, if one or more surgical instruments **I** is to be removed or added to the bundled or retained set of surgical instruments **I** the procedure outlined above for stringer attachment device **700** or alternate stringer attachment devices **800** may be utilized to add or remove surgical instruments **I**. Thus, surgical instrument support tray system **10** and stringer **100/A** reduces the time spent cleaning, sorting, counting, identifying and grouping surgical instruments **I,** extends the life expectancy of the surgical instruments, and enables thorough cleaning and decontamination of the surgical instruments.

In addition, surgical instrument support tray system **10** and stringer **100/A** preferably reduces the number of sharps being bundled or hidden with the surgical instruments **I** and being sent to sterile central.

In block or step **820** and **850,** surgical instruments **I** may be gathered together on one end of stringer **100/A,** stringer **100/A** may be contracted with retained parallel spaced and closed jaw surgical instruments **I,** and laid as a group of surgical instruments **I** on bottom **226** of tray bottom **220** and tray top **320** and may be positioned on tray bottom **220** wherein the grouped surgical instruments **I** or basket-like housing or enclosure with surgical instruments **I** in an organized and secure position and open jaw surgical may be wrapped, containerized (placed in a sterilization container, such as a basket with detachable lid) or stacked for sterile processing/distribution. Surgical instrument support tray(s) system **10** with stringer **100/A** retained parallel spaced and open jaw surgical instruments **I** may be sterilized (step **855**) in a commercial sterilizer using steam or other sterilant. The sterilant may access surgical instruments **I** via a plurality or set of holes such as apertures **240/340/440** in surgical instrument support tray system **10** to enable thorough cleaning and sterilization of surgical instruments. Once sterilized, surgical instrument support tray(s) system **10** with stringer **100** retained parallel spaced and open jaw surgical instruments **I** may be placed in inventory for future surgical use (step **860**).

The foregoing description and drawings comprise illustrative embodiments of the present invention. Having thus described exemplary embodiments, it should be noted by those ordinarily skilled in the art that the within disclosures are exemplary only, and that various other alternatives, adaptations, and modifications may be made within the scope of the present invention. Many modifications and other embodiments of the invention will come to mind to one ordinarily skilled in the art to which this invention pertains having the benefit of the teachings presented in the foregoing descriptions and the associated drawings. Although specific terms may be employed herein, they are used in a generic and descriptive sense only and not for purposes of limitation. Moreover, the present invention having been described in detail, it should be understood that various changes, substitutions and alterations can be made thereto without departing from the scope of the invention as defined by the appended claims. Accordingly, the present invention is not limited to the specific embodiments illustrated herein, but is limited only by the following claims.

## Claims

1. A surgical instrument support tray (10) configured to support one or more surgical instruments (I) having a pair of ring handles (R₁, R₂), the pair of ring handles (R₁, R₂) including a first ring handle (R₁)and a second ring handle (R₂), said surgical instrument support tray (10) comprising:
a surface (326) configured to support the one or more surgical instruments (I), and
a stringer (100A) having two or more rod sections (101), wherein a first rod section (101A) is configured to be inserted in the first ring handle (R₁) of the one or more surgical instruments (I) and a second rod section (101B) is configured to be inserted in the second ring handle (R₂) of the one or more surgical instruments (I),
**characterized in that**
said surface (326) further comprises two or more valleys (650) formed in parallel in said surface (326) and configured to receive the first ring handle (R₁) and the second ring handle (R₂) of the one or more surgical instruments (I); and
said stringer (100A) further comprises two or more angle sections (104A, 104B, 114A, 114B), each said angle section (104A, 104B, 114A, 114B) configured having one or more slidable connector sections (130C, 130D, 130E, 130F), each said one or more slidable connector sections (130C, 130D, 130E, 130F) configured to extend and retract along at least one of said two or more rod sections (101A, 101B), and two or more releasable connectors (130A, 130B), each said releasable connector (130A, 130B) positioned between two of said two or more angle sections (104A, 104B, 114A, 114B).

2. The surgical instrument support tray of claim 1, wherein said stringer (100A) is positioned with the pair of ring handles (R₁, R₂) of the surgical instruments (I) in two of said two or more valleys (650) to releasably retain the pair of ring handles (R₁, R₂) of the surgical instruments (I) therein,
wherein preferably said two or more valleys (650) form at least one pair of valleys (651, 652) configured to receive the pair of ring handles (R₁, R₂) of the surgical instrument (I),
wherein preferably said at least one pair of valleys (651, 652) are spaced apart, said at least one pair of valleys (651, 652) configured to releasably retain the surgical instruments (I) in an open position,
wherein preferably said at least one pair of valleys (651, 652) and said stringer (100A) are configured to maintain a spaced relationship between the surgical instruments (I).

3. The surgical instrument support tray of claim 1, wherein said surface (326) is configured with one or more perpendicular side walls (322, 323, 324, 325) extending therefrom to form a lid (320).

4. The surgical instrument support tray of claim 3, wherein said lid (320) is configured as a top for a container (220) of the surgical instruments (I).

5. The surgical instrument support tray of claim 4, wherein said lid (320) and said container (220) further comprise a plurality of holes (320) configured to enable sterilant to flow therethrough.

6. The surgical instrument support tray of claim 5, wherein at least one of said two or more angle sections (104A) further comprises a peg (104A.8, 104B.8, 114A.8, 114B.8).

7. The surgical instrument support tray of claim 6, wherein said peg (104A.8, 104B.8, 114A.8, 114B.8) is positioned perpendicular to said at least one of said two or more angle sections (104A), or
wherein said peg (104A.8, 104B.8, 114A.8, 114B.8) is releasably affixed to one of said plurality of holes (340).

8. The surgical instrument support tray of claim 6, wherein said peg (104A.8, 104B.8, 114A.8, 114B.8) is releasably affixed to one of said plurality of holes (340) and wherein one of said two or more rod sections (101) is configured parallel with one of said two or more valleys (650), or
wherein said peg (104A.8, 104B.8, 114A.8, 114B.8) is releasably affixed to one of said plurality of holes (340) and said two or more rod sections (101) are configured parallel with said two or more valleys (651, 652).

9. The surgical instrument support tray of claim 6, wherein said peg (104A.8, 104B.8, 114A.8, 114B.8) is releasably affixed to one of said plurality of holes (340), and at least one of said two or more rod sections (101) is configured parallel with one of said two or more valleys (650) and at least one other of said two or more rod sections (101) is configured nonparallel with one other of said two or more valleys (650).

10. The surgical instrument support tray of claim 5, wherein at least two of said two or more angle sections (104A, 104B) further comprises a peg (104A.8, 104B.8, 114A.8, 114B.8), and wherein said peg (104A.8, 104B.8, 114A.8, 114B.8) is releasably affixed to one of said plurality of holes (340) for said at least two of said two or more angle sections (104A, 104B), and at least one of said two or more rod sections (101A, 101B) is configured parallel or nonparallel with one of said two or more valleys (650).

11. The surgical instrument support tray of claim 5, further comprising a stringer attachment device (801) releasably affixed to said surface (326).

12. The surgical instrument support tray of claim 11, wherein said stringer attachment device (801) further comprises a snap-in clasp configured to releasably receive one of said two or more releasable connectors (130A, 130B),
wherein said clasp preferably further comprises a trough (802) to releasably receive one of said two or more releasable connectors (130A, 130B).

13. The surgical instrument support tray of claim 11, wherein said stringer attachment device (801) further comprises one or more pegs (808) configured to releasably affix said stringer attachment device (801) to said plurality of holes (340).

14. The surgical instrument support tray claim 11, wherein said stringer attachment device (801) is configured to enable a vertical position of said two or more rod sections (101A, 101B) of the stringer (100A) to string the surgical instruments (I) thereon.

15. The surgical instrument support tray of claim 5, wherein at least one of said two or more releasable connectors (130A, 130B) further comprises a protrusion (104B.7),
wherein preferably said protrusion (104B.7) is releasably affixed to one of said plurality of holes (340).

## Patentansprüche

1. Ablage- bzw. Trägerschale (10) für chirurgische Instrumente, die so konfiguriert ist, dass sie ein oder mehrere chirurgische Instrumente (I) mit einem Paar von Ringgriffen (R₁, R₂) trägt, wobei das Paar von Ringgriffen (R₁, R₂) einen ersten Ringgriff (R₁) und einen zweiten Ringgriff (R₂) aufweist, wobei die Trägerschale (10) für chirurgische Instrumente Folgendes aufweist:
eine Oberfläche (326), die so konfiguriert ist, dass sie ein oder mehrere chirurgische Instrumente (I) trägt, und
ein Auffädelelement bzw. einen Stringer (100A) mit zwei oder mehr Stab- bzw. Stangenabschnitten (101A), wobei ein erster Stangenabschnitt (101A) konfiguriert ist, um in den ersten Ringgriff (R₁) des einen oder der mehreren chirurgischen Instrumente (I) eingeführt zu werden, und wobei ein zweiter Stangenabschnitt (101B) konfiguriert ist, um in den zweiten Ringgriff (R₂) des einen oder der mehreren chirurgischen Instrumente (I) eingeführt zu werden,
**dadurch gekennzeichnet, dass**
die Oberfläche (326) ferner zwei oder mehr Mulden (650) aufweist, die parallel in der Oberfläche (326) ausgeformt sind und konfiguriert sind, den ersten Ringgriff (R₁) und den zweiten Ringgriff (R₂) des einen oder der mehreren chirurgischen Instrumente (I) aufzunehmen; und
dass der Stringer (100A) ferner zwei oder mehr Winkelabschnitte (104A, 104B, 114A, 114B) aufweist, wobei jeder Winkelabschnitt (104A, 104B, 114A, 114B) so konfiguriert ist, dass er ein oder mehrere verschiebbare Verbindungsabschnitte (130C, 130D, 130E, 130F) hat, wobei jeder des einen oder der mehreren verschiebbaren Verbindungsabschnitte (130C, 130D, 130E, 130F) konfiguriert ist, entlang des einen der zwei oder mehr Stangenabschnitte (101A, 101B) ausgefahren und zurückgezogen zu werden, und zwei oder mehr lösbare Verbinder (130A, 130B) aufweist, wobei jeder der lösbaren Verbinder (130A, 130B) zwischen zwei der zwei oder mehr Winkelabschnitte (104A, 104B, 114A, 114B) positioniert ist.

2. Trägerschale für chirurgische Instrumente nach Anspruch 1, wobei der Stringer (100A) mit dem Paar von Ringgriffen (R₁, R₂) der chirurgischen Instrumente (I) in zwei der zwei oder mehr Mulden (650) angeordnet ist, um das Paar von Ringgriffen (R₁, R₂) der chirurgischen Instrumente (I) lösbar darin zu halten,
wobei vorzugsweise die zwei oder mehr Mulden (650) zumindest ein Paar von Mulden (651, 652) formen, die so konfiguriert sind, dass sie das Paar von Ringgriffen (R₁, R₂) des chirurgischen Instrumentes (I) aufnehmen, wobei vorzugsweise das mindestens eine Paar von Mulden (651, 652) voneinander beabstandet ist, wobei das mindestens eine Paar von Mulden (651, 652) so konfiguriert ist, dass es die chirurgischen Instrumente (I) in einer offenen Position lösbar hält,
wobei vorzugsweise das mindestens eine Paar von Mulden (651, 652) und der Stringer (100A) so konfiguriert sind, dass sie eine beabstandete Beziehung zwischen den chirurgischen Instrumenten (I) aufrechterhalten.

3. Trägerschale für chirurgische Instrumente nach Anspruch 1, wobei die Oberfläche (326) mit einer oder mehreren senkrechten Seitenwänden (322, 323, 324, 325), die sich davon erstrecken, konfiguriert ist, um einen Deckel (320) zu formen.

4. Trägerschale für chirurgische Instrumente nach Anspruch 3, wobei der Deckel (320) als ein Oberteil für einen Behälter (220) der chirurgischen Instrumente (I) konfiguriert ist.

5. Trägerschale für chirurgische Instrumente nach Anspruch 4, wobei der Deckel (320) und der Behälter (220) ferner eine Vielzahl von Löchern (320) aufweisen, die konfiguriert sind, um zu ermöglichen, dass Sterilisationsmittel dort hindurch fließt.

6. Trägerschale für chirurgische Instrumente nach Anspruch 5, wobei zumindest einer der zwei oder mehr Winkelabschnitte (104A) ferner einen Stift (104A.8, 104B.8, 114A.8, 114B.8) aufweist.

7. Trägerschale für chirurgische Instrumente nach Anspruch 6, wobei der Stift (104A.8, 104B.8, 114A.8, 114B.8) senkrecht zu dem mindestens einen der zwei oder mehr Winkelabschnitte (104A) angeordnet ist, oder wobei der Stift (104A.8, 104B.8, 114A.8, 114B.8) lösbar an einem der Vielzahl von Löchern (340) befestigt ist.

8. Trägerschale für chirurgische Instrumente nach Anspruch 6, wobei der Stift (104A.8, 104B.8, 114A.8, 114B.8) lösbar an einem der Vielzahl von Löchern (340) befestigt ist und wobei einer der zwei oder mehr Stangenabschnitte (101) parallel zu einer der zwei oder mehr Mulden (650) konfiguriert ist, oder
wobei der Stift (104A.8, 104B.8, 114A.8, 114B.8) lösbar an einem der Vielzahl von Löchern (340) befestigt ist und wobei zwei oder mehr Stangenabschnitte (101) parallel zu den zwei oder mehr Mulden (651, 652) konfiguriert sind.

9. Trägerschale für chirurgische Instrumente nach Anspruch 6, wobei der Stift (104A.8, 104B.8, 114A.8, 114B.8) lösbar an einem der Vielzahl von Löchern (340) befestigt ist und wobei zumindest einer der zwei oder mehr Stangenabschnitte (101) parallel zu einer der zwei oder mehr Mulden (650) konfiguriert ist, und zumindest ein anderer der zwei oder mehr Stangenabschnitte (101) nicht parallel zu einer anderen der zwei oder mehr Mulden (650) konfiguriert ist.

10. Trägerschale für chirurgische Instrumente nach Anspruch 5, wobei mindestens zwei der zwei oder mehr Winkelabschnitte (104A, 104B) ferner einen Stift (104A.8, 104B.8, 114A.8, 114B.8) aufweist und wobei der Stift (104A.8, 104B.8, 114A.8, 114B.8) lösbar an einem der Vielzahl von Löchern (340) für die mindestens zwei der zwei oder mehr Winkelabschnitte (104A, 104B) befestigt ist und wobei mindestens einer der zwei oder mehr Stangenabschnitte (101A, 101B) parallel oder nicht parallel zu einer der zwei oder mehr Mulden (650) konfiguriert ist.

11. Trägerschale für chirurgische Instrumente nach Anspruch 5, welches ferner eine Stringer-Befestigungsvorrichtung (801) aufweist, die lösbar an der Oberfläche (326) befestigt ist.

12. Trägerschale für chirurgische Instrumente nach Anspruch 11, wobei die Stringer-Befestigungsvorrichtung (801) ferner einen Einrastverschluss aufweist, der konfiguriert ist, um lösbar einen der zwei oder mehr lösbaren Verbinder (130A, 130B) aufzunehmen,
wobei der Verschluss vorzugsweise ferner eine Rinne (802) aufweist, um einen der zwei oder mehr lösbaren Verbinder (130A, 130B) lösbar aufzunehmen.

13. Trägerschale für chirurgische Instrumente nach Anspruch 11, wobei die Stringer-Befestigungsvorrichtung (801) ferner einen oder mehrere Stifte (808) aufweist, der bzw. die konfiguriert sind, um die Stringer-Befestigungsvorrichtung (801) an der Vielzahl von Löchern (340) zu befestigen.

14. Trägerschale für chirurgische Instrumente nach Anspruch 11, wobei die Stringer-Befestigungsvorrichtung (801) konfiguriert ist, um eine vertikale Position der zwei oder mehr Stangenabschnitte (101A, 101B) des Stringers (100A) zu ermöglichen, um die chirurgischen Instrumente (I) darauf aufzufädeln bzw. aufzureihen.

15. Trägerschale für chirurgische Instrumente nach Anspruch 5, wobei mindestens eine der zwei oder mehr lösbaren Verbinder (130A, 130B) ferner einen Vorsprung (104B.7) aufweist,
wobei der Vorsprung (104B.7) vorzugsweise lösbar an einem der Vielzahl von Löchern (340) befestigt ist.

## Revendications

1. Plateau support d'instruments chirurgicaux (10) agencé pour supporter un ou plusieurs instruments chirurgicaux (I) comportant une paire de poignées en anneau (R₁, R₂), la paire de poignées en anneau (R₁, R₂) comprenant une première poignée en anneau (R₁) et une deuxième poignée en anneau (R₂), le plateau support d'instruments chirurgicaux (10) comprenant :
une surface (326) agencée pour supporter lesdits un ou plusieurs instruments chirurgicaux (I), et
un longeron (100A) comportant deux ou plusieurs sections de tige (101), dans lesquelles une première section de tige (101A) est agencée pour être insérée dans la première poignée en anneau (R₁) desdits un ou plusieurs instruments chirurgicaux (I) et une deuxième section de tige (101B) est agencée pour être insérée dans la deuxième poignée en anneau (R₂) desdits un ou plusieurs instruments chirurgicaux (I),
**caractérisé en ce que**
ladite surface (326) comprend en outre deux ou plusieurs parties en creux (650) formées parallèles dans ladite surface (326) et agencées pour recevoir la première poignée en anneau (R₁) et la deuxième poignée en anneau (R₂) desdits un ou plusieurs instruments chirurgicaux (I) ; et
le longeron (100A) comprenant en outre deux ou plusieurs sections d'angle (104A, 104B, 114A, 114B), chaque section d'angle (104A, 104B, 114A, 114B) étant munie d'une ou plusieurs sections de connecteur pouvant coulisser (130C, 130D, 130E, 130F), chacune desdites une ou plusieurs sections de connecteur pouvant coulisser (130C, 130D, 130E, 130F) étant agencée pour s'étirer et se rétracter le long d'au moins l'une desdites deux ou plusieurs sections de tige (101A, 101B), et deux ou plusieurs connecteurs libérables (130A, 130B), chaque connecteur libérable (130A, 130B) étant positionné entre deux desdites deux ou plusieurs sections d'angle (104A, 104B, 114A, 114B).

2. Plateau support d'instruments chirurgicaux selon la revendication 1, dans lequel le longeron (100A) est positionné avec la paire de poignées en anneau (R₁, R₂) des instruments chirurgicaux (I) dans deux desdites deux ou plusieurs parties en creux (650) pour retenir de façon libérable la paire de poignées en anneau (R₁, R₂) des instruments chirurgicaux (I),
dans lequel de préférence lesdites deux ou plusieurs parties en creux (650) forment au moins une paire de parties en creux (651, 652) agencée pour recevoir la paire de poignées en anneau (R₁, R₂) des instruments chirurgicaux (I),
dans lequel de préférence les parties en creux de ladite au moins une paire de parties en creux (651, 652) sont espacées, lesdites au moins deux parties en creux (651, 652) étant agencées pour retenir de façon libérable les instruments chirurgicaux (I) dans une position ouverte,
dans lequel de préférence lesdites au moins deux parties en creux (651, 652) et le longeron (100A) sont agencés pour maintenir une relation d'espacement entre les instruments chirurgicaux (I).

3. Plateau support d'instruments chirurgicaux selon la revendication 1, dans lequel ladite surface (326) est munie d'une ou plusieurs parois latérales perpendiculaires (322, 323, 324, 325) s'étendant à partir de celle-ci pour former un couvercle (320).

4. Plateau support d'instruments chirurgicaux selon la revendication 3, dans lequel le couvercle (320) est agencé sous forme de la partie supérieure d'un conteneur (220) des instruments chirurgicaux (I).

5. Plateau support d'instruments chirurgicaux selon la revendication 4, dans lequel le couvercle (320) et le conteneur (220) comprennent en outre une pluralité de trous (320) agencés pour permettre à un liquide de stérilisation de circuler à travers ceux-ci.

6. Plateau support d'instruments chirurgicaux selon la revendication 5, dans lequel au moins l'une desdites deux ou plusieurs sections d'angle (104A) comprend en outre une cheville (104A.8, 104B.8, 114A.8, 114B.8).

7. Plateau support d'instruments chirurgicaux selon la revendication 6, dans lequel la cheville (104A.8, 104B.8, 114A.8, 114B.8) est positionnée perpendiculairement à ladite au moins une des deux ou plusieurs sections d'angle (104A), ou
dans lequel la cheville (104A.8, 104B.8, 114A.8, 114B.8) est fixée de façon amovible à l'un de la pluralité de trous (340).

8. Plateau support d'instruments chirurgicaux selon la revendication 6, dans lequel la cheville (104A.8, 104B.8, 114A.8, 114B.8) est fixée de façon amovible à l'un de la pluralité de trous (340) et dans lequel l'une des deux ou plusieurs sections de tige (101) est agencée parallèle à l'une des deux ou plusieurs parties en creux (650), ou
dans lequel la cheville (104A.8, 104B.8, 114A.8, 114B.8) est fixée de façon amovible à l'un de la pluralité de trous (340) et les deux ou plusieurs sections de tige (101) sont agencées parallèles aux deux ou plusieurs parties en creux (651, 652).

9. Plateau support d'instruments chirurgicaux selon la revendication 6, dans lequel la cheville (104A.8, 104B.8, 114A.8, 114B.8) est fixée de manière amovible à l'un de la pluralité de trous (340), et au moins l'une des deux ou plusieurs sections de tige (101) est agencée parallèle à l'une des deux ou plusieurs parties en creux (650) et au moins une autre des deux ou plusieurs sections de tige (101) est agencée non parallèle à une autre des deux ou plusieurs parties en creux (650).

10. Plateau support d'instruments chirurgicaux selon la revendication 5, dans lequel au moins deux desdites deux ou plusieurs sections d'angle (104A, 104B) comprend en outre une cheville (104A.8, 104B.8, 114A.8, 114B.8), et dans lequel la cheville (104A.8, 104B.8, 114A.8, 114B.8) est fixée de façon amovible à l'un de la pluralité de trous (340) pour lesdites au moins deux des deux ou plusieurs sections d'angle (104A, 104B), et au moins l'une des deux ou plusieurs sections de tige (101A, 101B) est agencée parallèle ou non parallèle à l'une des deux ou plusieurs parties en creux (650).

11. Plateau support d'instruments chirurgicaux selon la revendication 5, comprenant en outre un dispositif de fixation de longeron (801) fixé de façon amovible à ladite surface (326).

12. Plateau support d'instruments chirurgicaux selon la revendication 11, dans lequel le dispositif de fixation de longeron (801) comprend en outre un fermoir à encliquetage agencé pour recevoir de façon amovible l'un des deux ou plusieurs connecteurs libérables (130A, 130B),
dans lequel le fermoir comprend en outre de préférence une dépression (802) pour recevoir de façon amovible l'un des deux ou plusieurs connecteurs libérables (130A, 130B).

13. Plateau support d'instruments chirurgicaux selon la revendication 11, dans lequel le dispositif de fixation de longeron (801) comprend en outre une ou plusieurs chevilles (808) agencées pour fixer de façon libérable le dispositif de fixation de longeron (801) à la pluralité de trous (340).

14. Plateau support d'instruments chirurgicaux selon la revendication 11, dans lequel le dispositif de fixation de longeron (801) est agencé pour permettre une position verticale des deux ou plusieurs sections de tige (101A, 101B) du longeron (100A) pour enfiler en série les instruments chirurgicaux (I) dessus.

15. Plateau support d'instruments chirurgicaux selon la revendication 5, dans lequel au moins l'un des deux ou plusieurs connecteurs libérables (130A, 130B) comprend en outre une protubérance (104B.7),
dans lequel de préférence la protubérance (104B.7) est fixée de façon libérable à l'un de la pluralité de trous (340).
